# EUROPEAN PATENT APPLICATION

(11) **EP 4 361 136 A1**
(43) Date of publication of application: **01.05.2024**
(21) Application number: 22824361.4
(22) Date of filing: 17.06.2022
(51) Int. Cl.: C07D 313/00, A61K 31/365, A61P 35/00

(54) **COMPOUND FOR THE TREATMENT OF GLIOBLASTOMA**

(30) Priority: 18.06.2021 ES 202130566
(71) Applicant: Universidad De Córdoba, 14005 Córdoba (ES); Servicio Andaluz de Salud, 41071 Sevilla (ES)
(72) Inventor: FUENTES FAYOS, Antonio Carlos, 14004 Córdoba (ES); LUQUE HUERTAS, Raúl Miguel, 14004 Córdoba (ES); JIMÉNEZ VACAS, Juan Manuel, 14004 Córdoba (ES); SOLIVERA VELA, Juan, 14004 Córdoba (ES); BLANCO ACEVEDO, Cristóbal, 14004 Córdoba (ES); CASTAÑO FUENTES, Justo P., 14004 Córdoba (ES); GAHETE ORTIZ, Manuel D., 14004 Córdoba (ES); GARCÍA GARCÍA, Miguel Eduardo, 14004 Córdoba (ES)
(74) Representative: San Martin Alarcia, Esther
(86) International application number: PCT/ES2022/070386
(87) International publication number: WO 2022/263702

(57) **Abstract**

Pladienolide-b for use in the treatment of glioblastoma.

## Description

The present invention is within the field of medicine and relates to compounds for the treatment of high-grade astrocytoma. Compositions of these compounds and combined preparations, along with other active ingredients, are also provided.

### STATE OF THE ART

Splicing is the process by which the coding and non-coding regions of a gene are restructured and rearranged to generate a variety of RNA transcripts, resulting in the emergence of different variants and isoforms of proteins which, in many cases, exert distinct and even opposite biological functions. Alteration of the splicing machinery can lead to the generation of aberrant splicing variants, which have been linked to the development and/or progression of several diseases, including cancer.

In particular, the importance of the spliceosome in the pathophysiology of metabolic diseases and cancer has recently been highlighted due to its versatility in generating aberrant alternative splicing variants.

Aberrant splicing variants have been directly associated with development and progression of tumours as a result of altered splicing and quality control of mRNA. In this sense, recent studies indicate that the cellular machinery that controls the splicing process, also known as spliceosome, is altered in different types of tumours giving rise to this aberrant splicing that gives rise to variants of different genes (*GFAP3, VEGF4, TP535, BCL2L16,* etc.) with different oncogenic implications. This alteration is mainly caused by the presence, alteration or loss of relevant components of this key molecular machinery that is in charge of properly processing pre-mRNA to obtain mature mRNA that regulates cell physiology. Recently, several components of the spliceosome, such as the splicing factor SRSF3, have been identified as oncogenic inducers in the pathophysiology of brain tumours. Malignant brain tumours are the most lethal tumours in patients under 40 years of age and have a survival rate of no more than 14 months after diagnosis. In particular, glioblastomas, the most aggressive brain tumour, have no effective anti-tumour treatment causing the greatest loss of patients compared to other more prevalent but less deadly cancers. In addition, these tumours have a high rate of post-surgical recurrence and resistance to treatment. Therefore, further exploration of the spliceosome as a source of molecules susceptible to being targets of new drugs could provide a novel approach to address malignant brain tumours, moving away from the standard ineffective drug targets currently used against glioblastoma.

### DESCRIPTION OF THE FIGURES

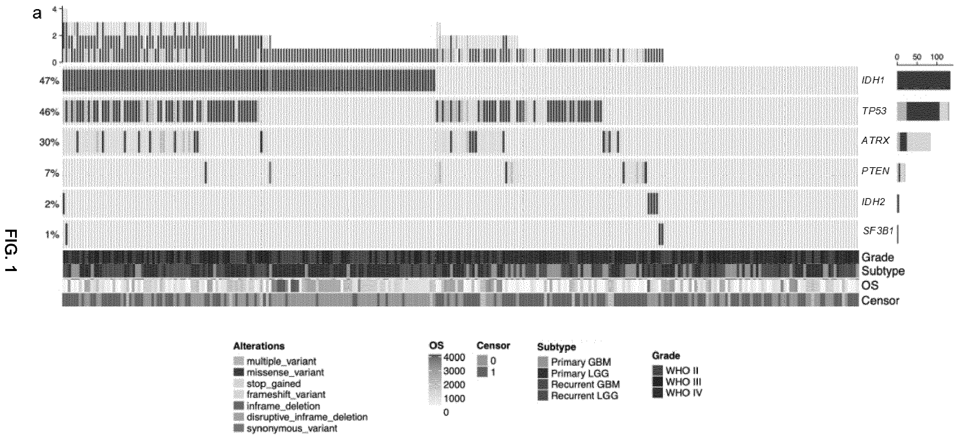

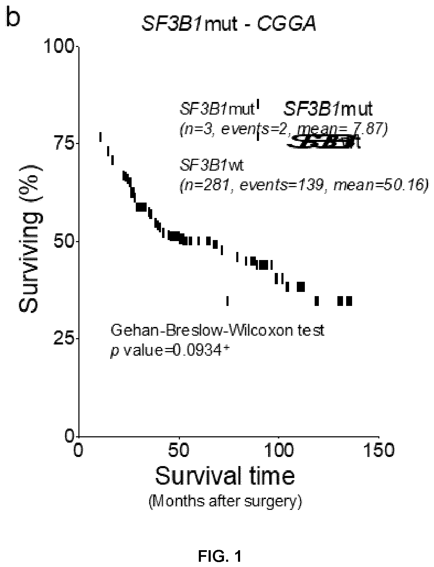

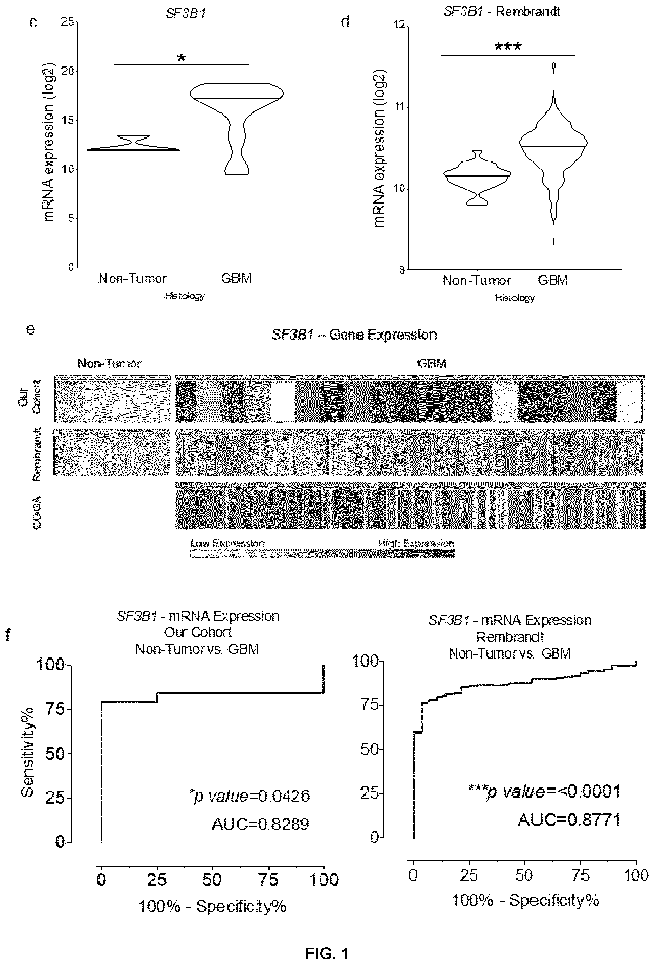

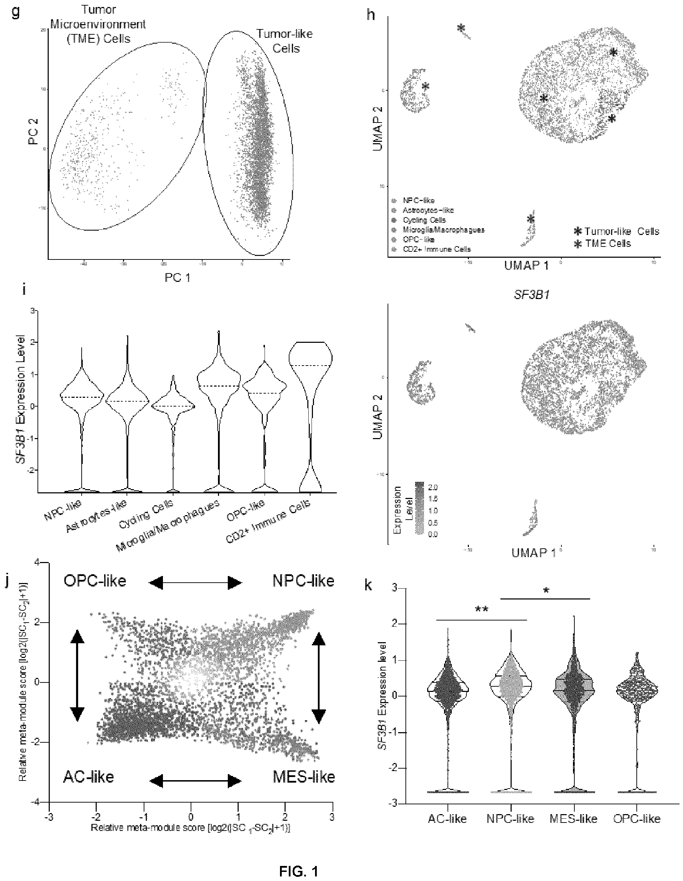

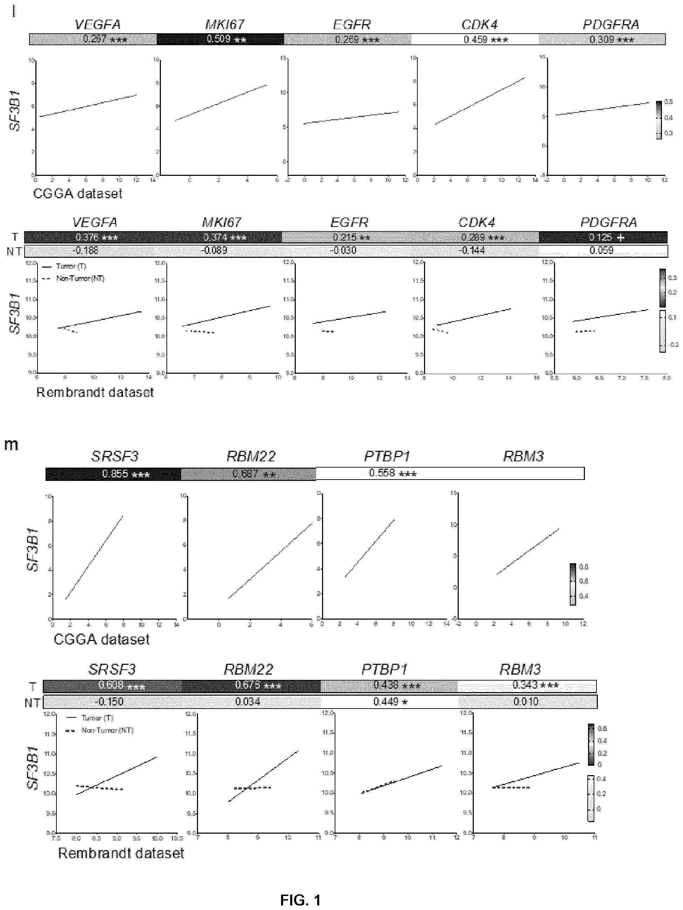

**Figure 1.** *SF3B1* is mutated and markedly overexpressed in human GBM samples compared to non-tumour brain samples. (a) Somatic mutations *of IDH1, TP53, ATRX, PTEN, IDH2* and *SF3B1* in glioma samples (n = 284) from the CGGA dataset. (b) Kaplan-Meier survival curves for SF3B1-mutated and wild-type glioma patients from the CGGA dataset (Gehan-Breslow-Wilcoxon test, p = 0.0934 +). (c) *SF3B1* mRNA levels in control and glioblastoma (GBM) samples in our patient cohort and in the external Rembrandt cohort (d). (e) Non-hierarchical heatmap generated comparing *SF3B1* expression levels in control brain tissues and/or GBM samples using our cohort, Rembrandt cohorts, and CGGA. (f) Receptor operating characteristic (ROC) curve: analysis of *SF3B1* expression curve using control and GBM samples from our cohort and external Rembrandt cohorts. Single cell level characterization of *SF3B1* across intratumoural human cell populations: (g) Principal component analysis discriminating tumour microenvironment (TME) cells from tumour-like cells of the single cell dataset. (h) Distribution of *SF3B1* expression in a distinct Uniform Manifold Approximation and Projection (UMAP) cluster (left panel: matching UMAP clusters with intratumour cell subtypes; right panel: UMAP feature plot showing *SF3B1* expression). (i) *SF3B1* expression across different identified intratumoural cell subtypes. (j) Glioblastoma cells sorted by transcriptional programs in bidimensional representation using the relative metamodule score [log2 (| SC1-SC2 | +1)]. Each quadrant corresponds to a cellular state. (k) *SF3B1* expression across different transcriptional programs of GBM cells. Correlation of *SF3B1* with different key prognostic biomarkers (I) and relevant spliceosome components (m) in GBM samples from CGGA (top panel) and Rembrandt (bottom panel) cohorts, with non-tumour samples for the Rembrandt dataset.

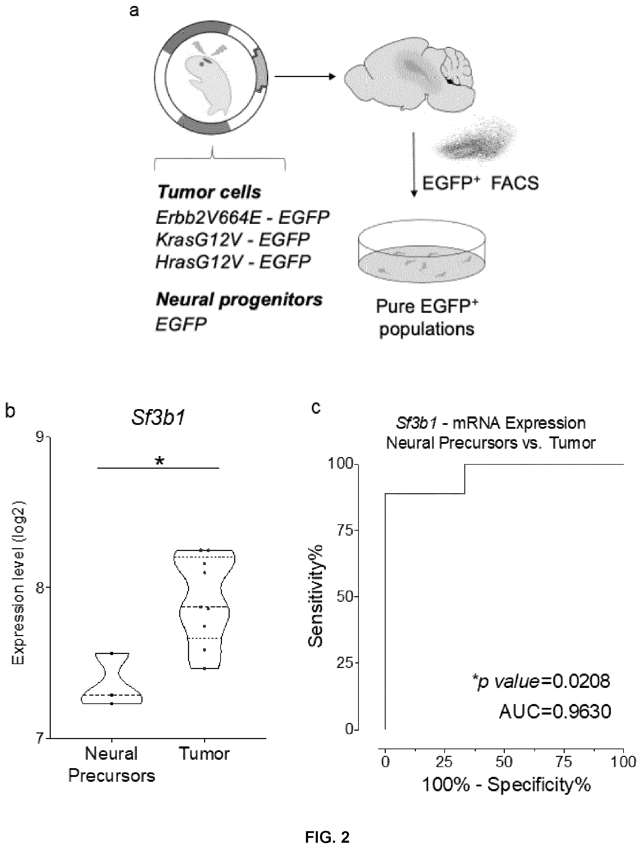

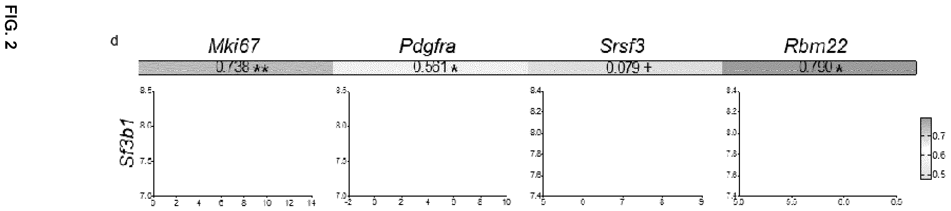

**Figure 2.** *Sf3b1* is overexpressed in different mouse models with glioma. (a) Generation of glioblastoma mouse models (GBM) by injection of plasmid DNA mixture into the left lateral ventricle after mouse brain electroporation (EP) (adapted Breunig et al., 2016). (b) mRNA expression levels, and (c) ROC curve analysis of *Sf3b1* in control and tumour samples from the electroporated mouse model. (d) Correlation of *Sf3b1* with different key prognostic biomarkers and relevant spliceosome components in GBM samples from these models.

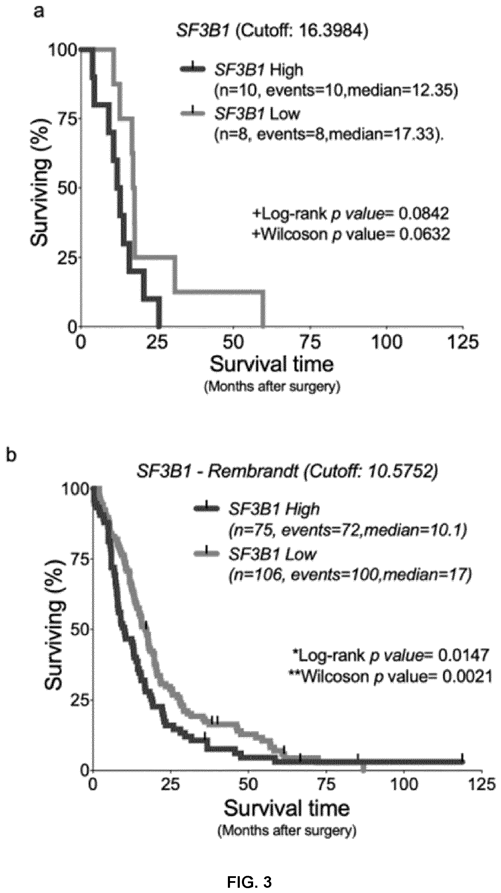

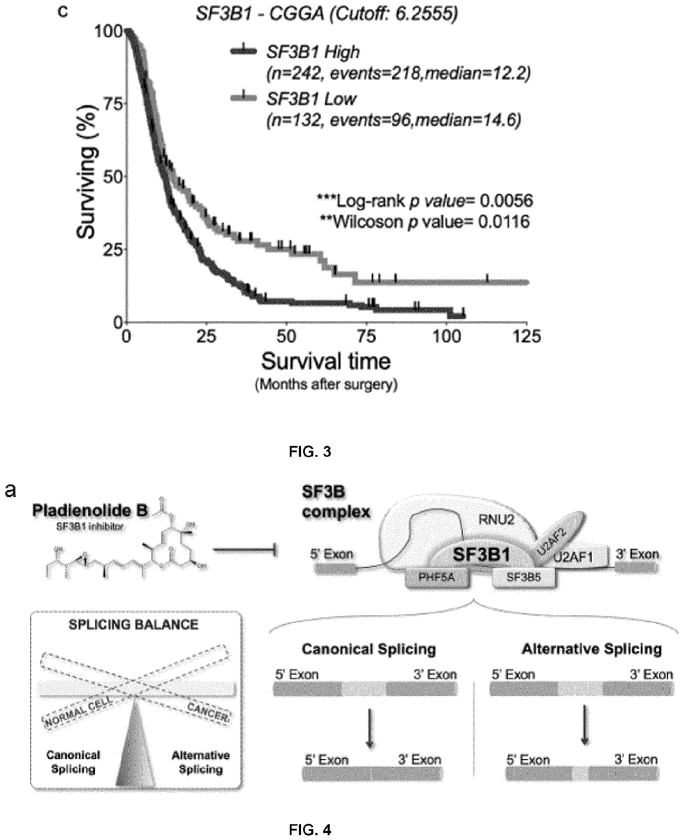

**Figure 3.** *SF3B1* overexpression is associated with a low survival rate. Kaplan-Meier survival curves of patients with glioblastoma (GBM) dividing patients according to high or low *SF3B1* expression levels in our cohort (a), as well as in the Rembrandt (b) and CGGA (c) datasets.

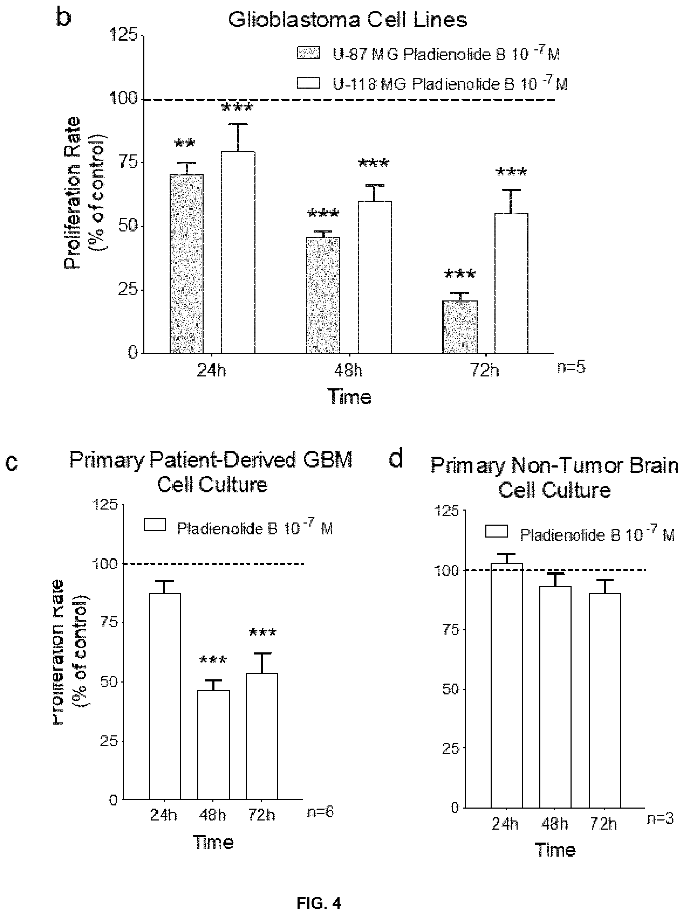

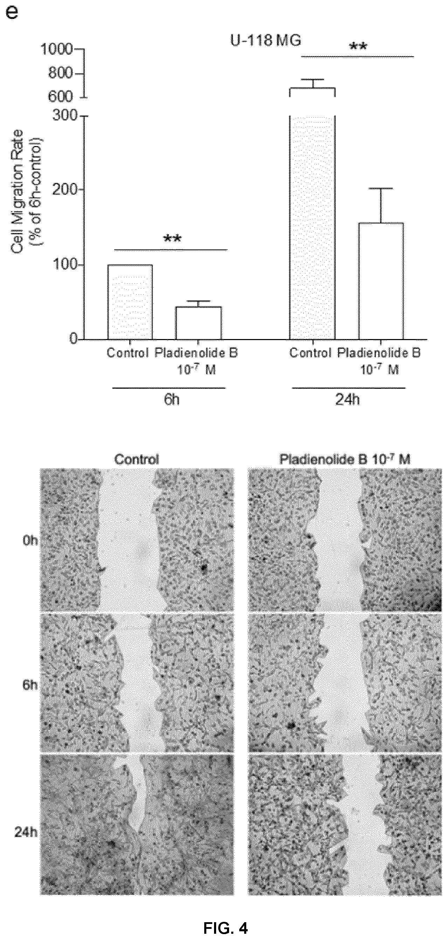

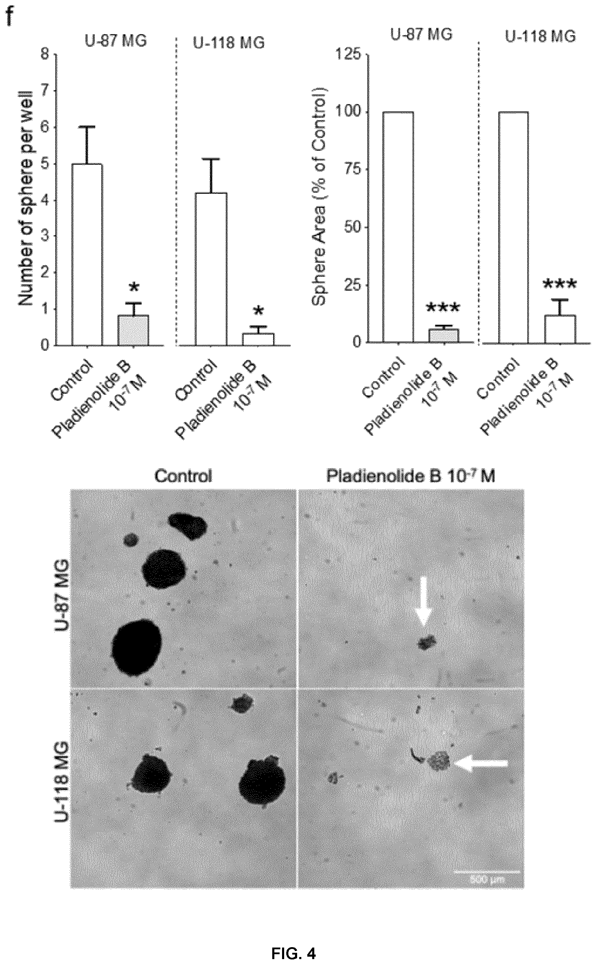

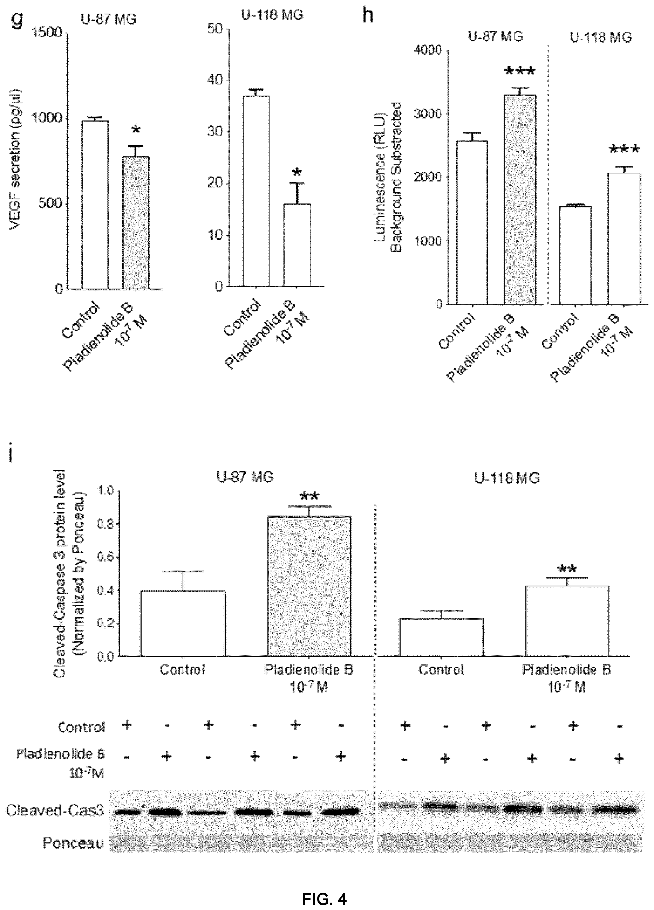

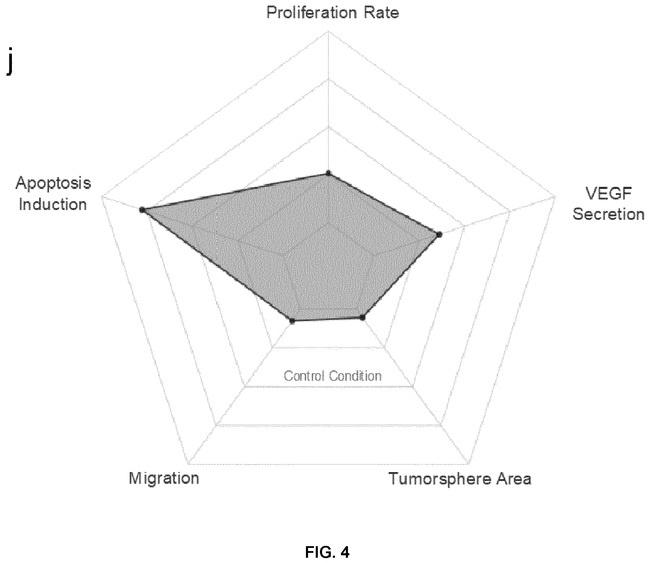

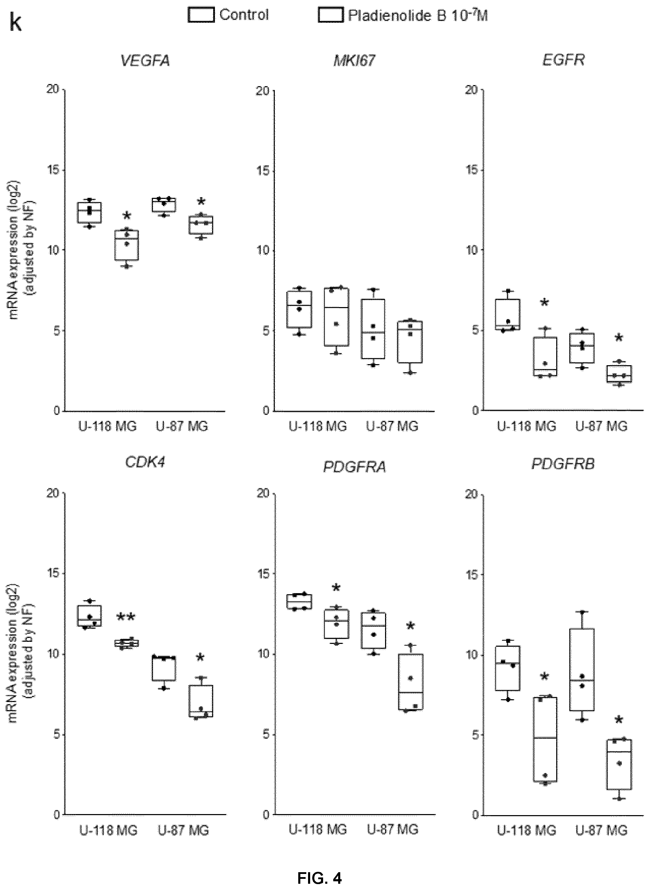

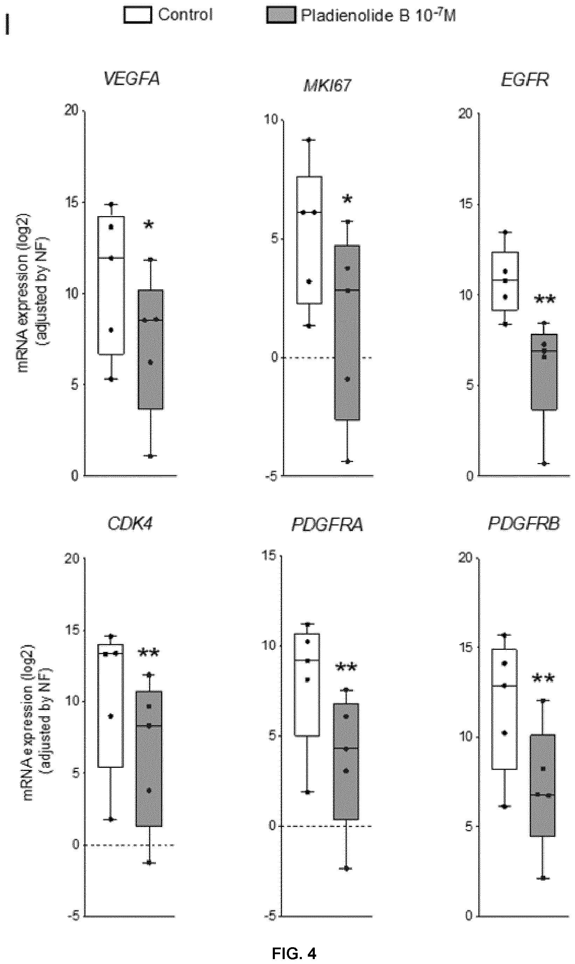

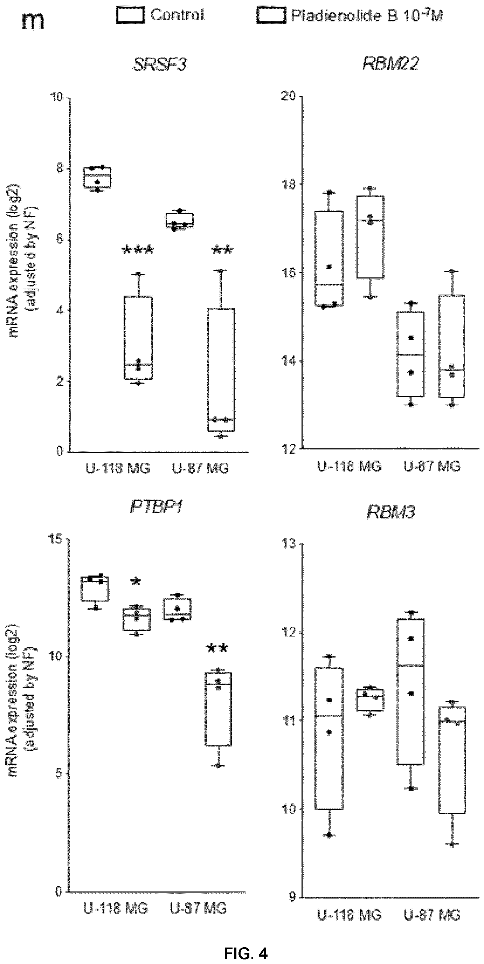

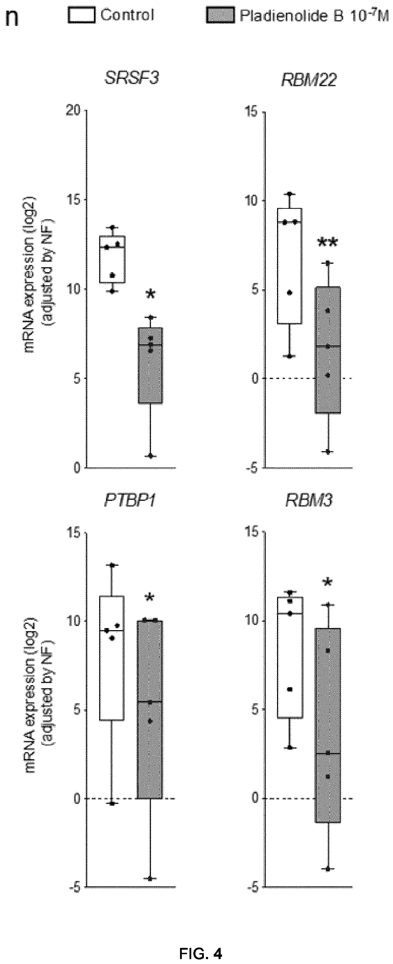

**Figure 4.** Pharmacological inhibition of SF3B1 with pladienolide-b *in vitro* decreases critical functional parameters of aggressiveness and key markers of tumour development/progression/aggressiveness in glioblastoma (GBM) cells compared to control conditions. (a) Schematic representation of the effect of SF3B1 inhibitor, pladienolide-b. Proliferation rate in response to pladienolide-b administration in human GBM cell lines (U-87 MG and U-118 MG; n = 5) (b), in patient-derived primary GBM cells (n = 6) (c), and in non-tumour primary brain cell culture (n = 3) (d). (e) Migration rate after pladienolide-b administration in U-118 MG cells (representative images of migration capacity are also included; n = 5). (f) Tumoursphere formation assay showing sphere area and number of tumourspheres per well in response to pladienolide-b administration in U-87 MG and U-118 MG cells (n = 3; representative images of tumoursphere formation are also included). (g) VEGF secretion in response to pladienolide-b in U-87 MG and U-118 MG cells (n = 3). (h) Apoptosis rate after pladienolide-b administration in U-87 MG and U-118 MG cells (n = 3). (i) Levels of cleaved caspase-3 protein after 24h incubation with pladienolide-b determined by Western blot (n = 3). (j) Summary of the effect of pladienolide-b through the different functional parameters mentioned above. Expression of different tumour progression markers after pladienolide-b treatment in the two GBM cell lines (k) and primary patient-derived GBM cells (I). Expression of different oncogenic biomarkers of spliceosome components after pladienolide-b treatment in the two GBM cell lines (m) and in primary patient-derived GBM cells (n).

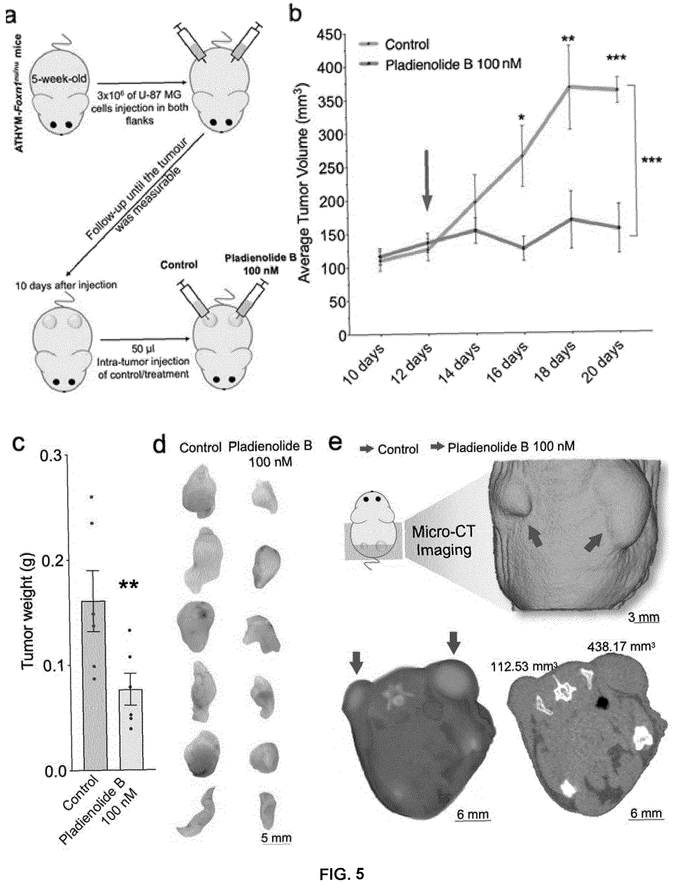

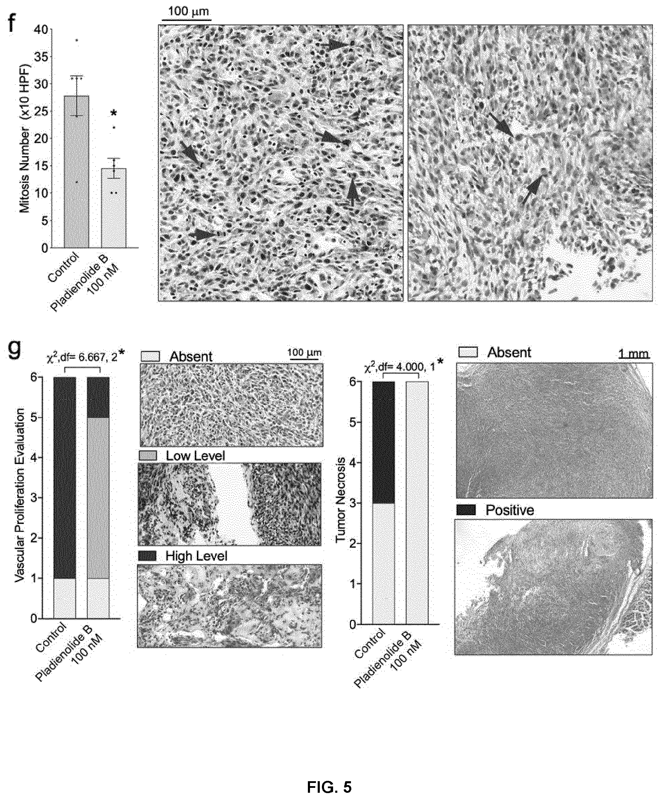

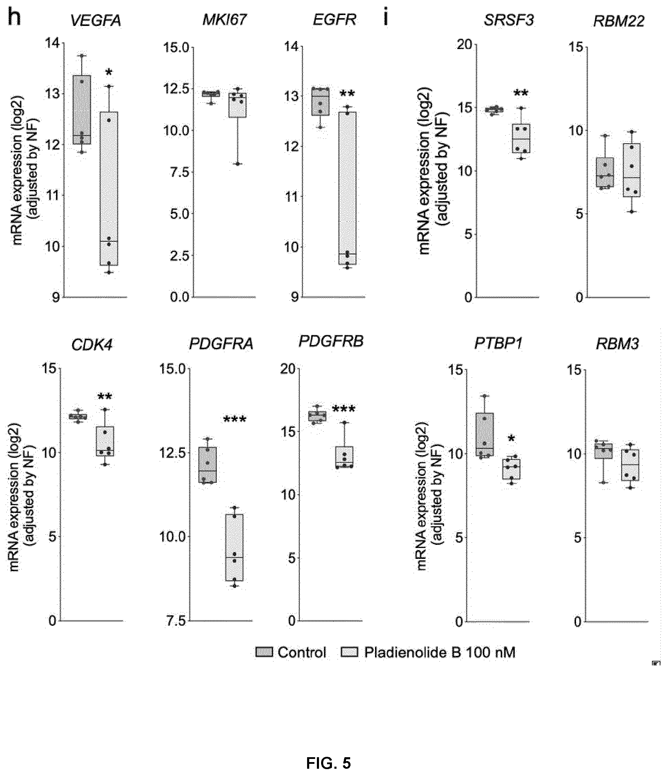

**Figure 5.** *In vivo* pharmacological inhibition of SF3B1 with pladienolide-b alters glioblastoma (GBM) progression and vascularization. (a) Generation of a preclinical xenograft GBM model by inoculation of U-87 MG cells (n = 6). Average tumour volume (b) and weight (c) after pladienolide-b injection intratumorally versus control treated tumours. The green arrow in (b) indicates the corresponding treatment time (intratumoral injection with pladienolide-b or control). (d) Images of each tumour at the time of sacrifice are shown individually. (e) 2D and 3D Micro-CT images of a representative pre-clinical xenograft GBM model. (f) Number of mitoses (x10 HPF; left panel) and representative H&E staining images (right panel) comparing intratumoural injection of pladienolide-b versus control treated tumour samples. (g) Assessment of vascular proliferation and representative H&E staining images (left panel) as well as assessment of tumour necrosis and representative H&E staining images (right panel) of intratumoural pladienolide-b injection versus control treated tumour samples. All these assessments were determined by experienced pathologists. Expression of different markers of tumour progression (h) and biomarkers of oncogenic spliceosome components (i) after pladienolide-b treatment in the preclinical GBM xenograft model.

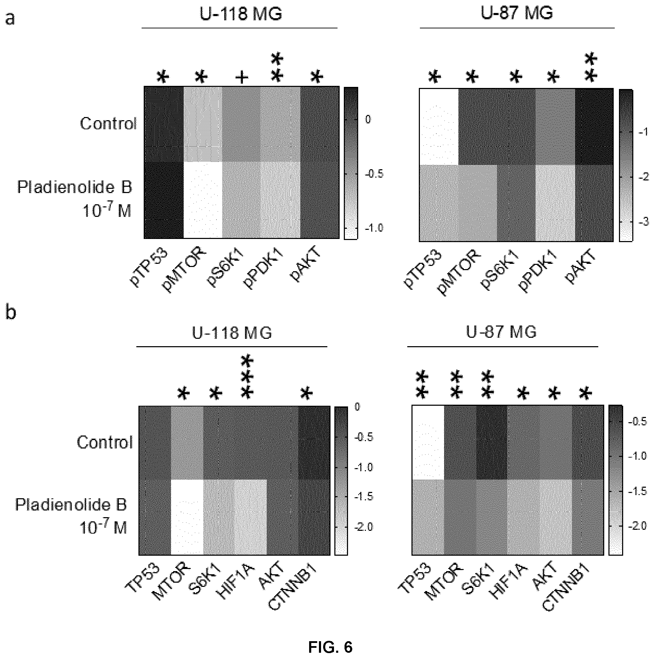

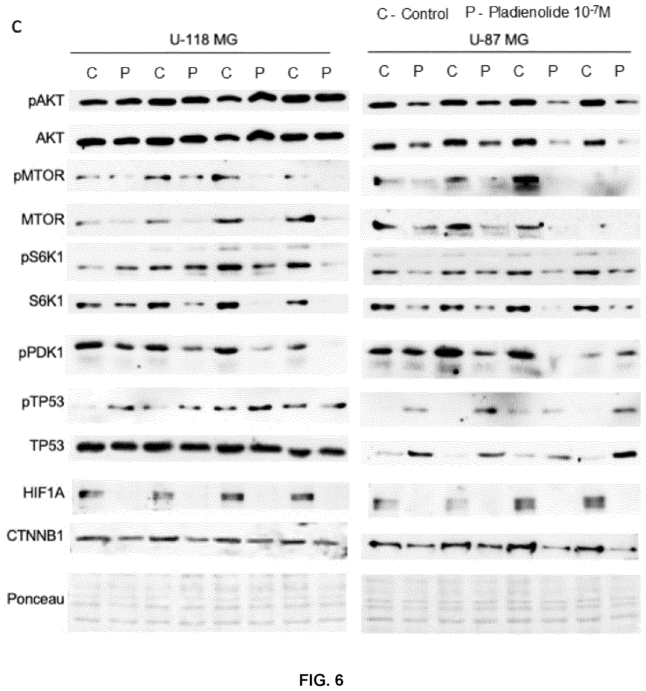

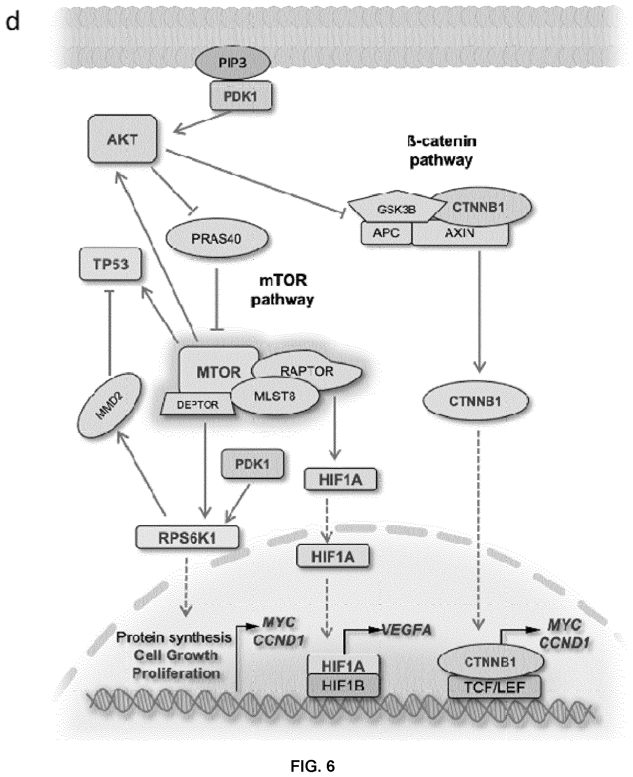

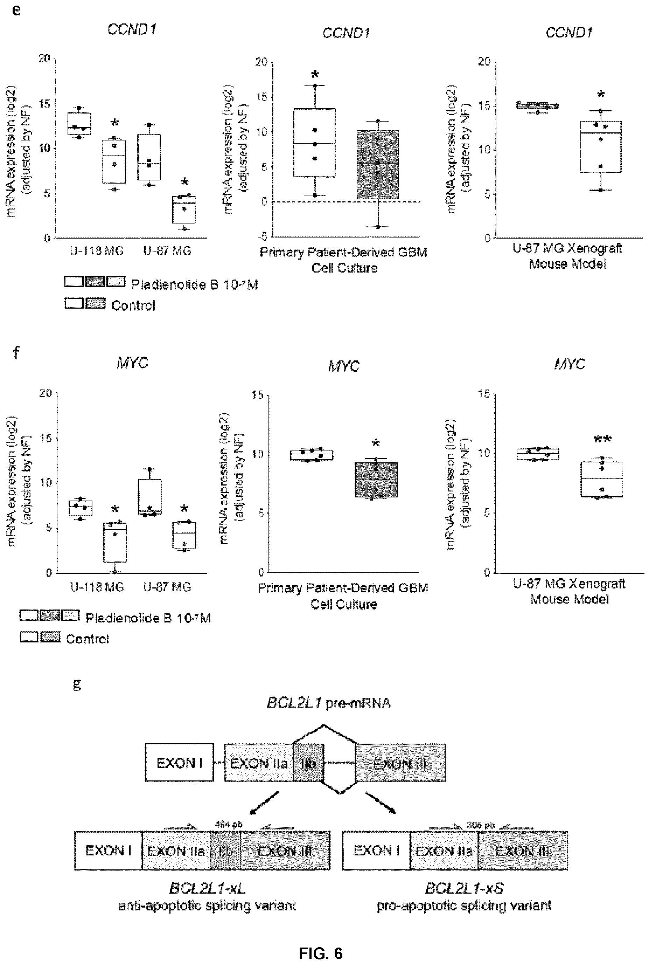

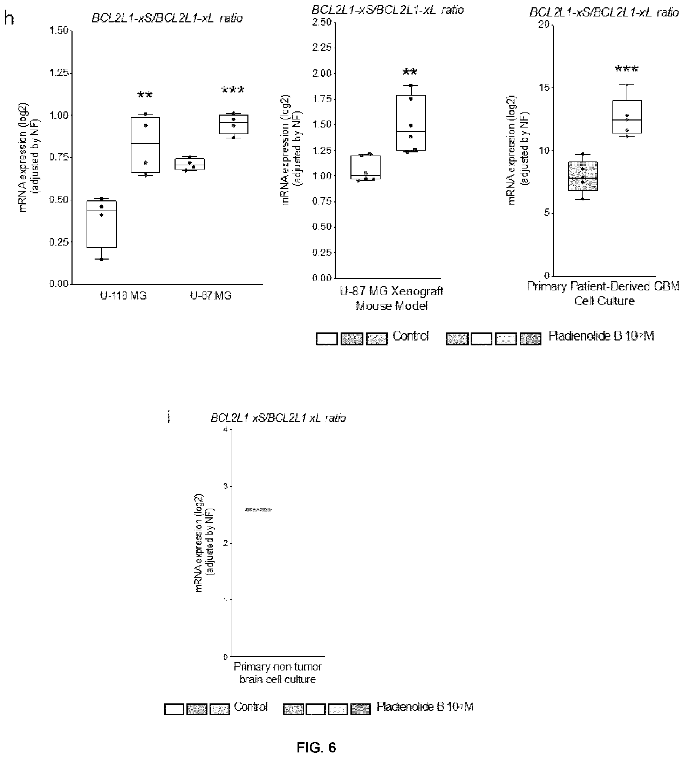

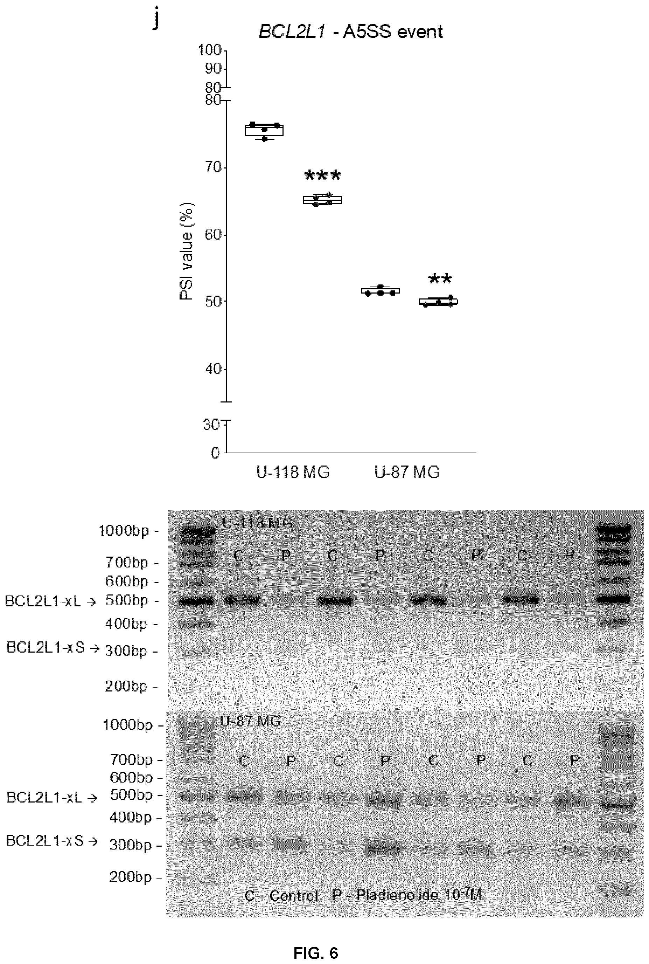

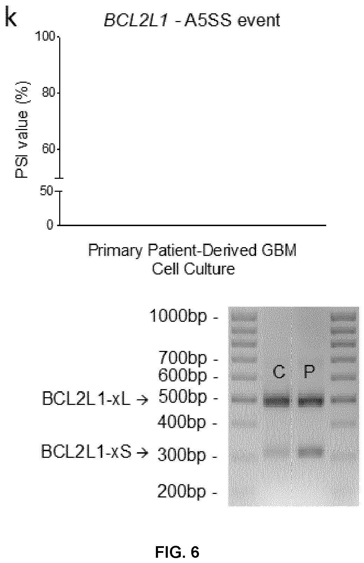

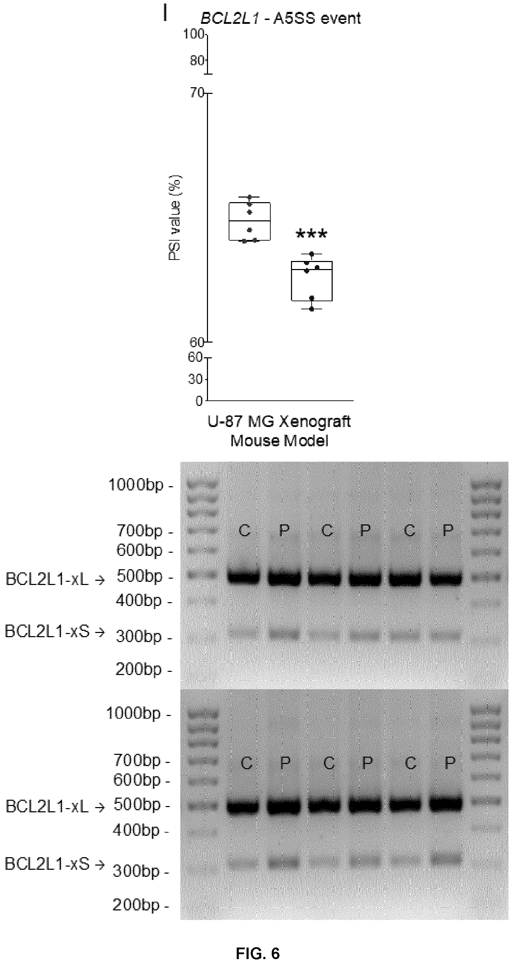

**Figure 6.** The pharmacological block of SF3B1 reveals AKT-mTOR and ß-catenin signaling pathways and alternative splicing of BCL2L1 as the main drivers of the antitumour effects of pladienolide-b in glioblastomas (GBM). (a) Heatmap showing phosphorylated protein level and (b) total protein levels of various components of the AKT/mTOR and ß-catenin pathways in GBM cells (U-87 MG and U-118 MG) after pladienolide-b administration. (c) Images of Western blot results shown in (a) and (b). (d) Diagram of AKT-MTOR and ß-catenin pathways showing the down-regulated (red) and up-regulated (green) components/processes after pladienolide-b administration identified in this work. CCND1 (e) and MYC (f) mRNA levels as assessment criteria of the AKT/mTOR and ß-catenin pathways in GBM cells (U-87 MG and U-118 MG), patient-derived primary GBM cells and in a preclinical GBM model after pladienolide-b administration. (g) BCL2L1 splicing variants produced by a splicing event at the 5'alternative splicing site (5'SS) and associated with the apoptosis and cell death pathway. (h) BCL2L1-xS / BCL2L1-xL ratio determined by qPCR in GBM cell lines (U-87 MG and U-118 MG), in the preclinical GBM model and in patient-derived primary GBM cells in response to pladienolide-b treatment. (i) BCL2L1-xS / BCL2L1-xL ratio determined by qPCR in cultured primary non-tumour brain cells after pladienolide-b administration. PSI of the BCL2L1 5'SS event in GBM cell lines (U-87 MG and U-118 MG), (j) in patient-derived primary GBM cells, (k) and in the preclinical GBM xenograft model (I) in response to pladienolide-b treatment.

### DESCRIPTION OF THE INVENTION

Splicing factor 3B subunit 1 (SF3B1) is an essential central component of the spliceosome being the largest subunit of the SF3B complex. SF3B1 together with splicing factor 3a, U2AF and a 12S RNA constitute the U2 small core ribonucleoprotein complex (U2-snRNP complex) that binds to the pre-mRNA upstream of the intron branching site, being a key step in the formation and functionality of the splicing core. Furthermore, this component of the spliceosome is essential in the complex formed together with U12, being crucial in both the major and minor spliceosomes. SF3B1 is one of several spliceosome components whose gene has been identified as frequently mutated in numerous cancers such as myelodysplastic syndrome, breast cancer, prolactinomas, uveal melanoma, and pancreatic ductal adenocarcinoma.

However, the oncogenic involvement of this component of the spliceosome, its somatic mutations, and expression profile have not been characterized in glioblastomas nor its association with molecular features and clinical parameters.

As shown in the examples of the present invention, the splicing factor SF3B1 is markedly mutated and widely overexpressed in glioblastoma samples compared to non-tumour samples in different glioblastoma models (see Figure 1 and 2) and is associated with molecular/clinical features such as patient survival (Figure 3).

Specifically, the authors of the present invention evaluated antitumour actions using a specific inhibitor of SF3B1, pladienolide B, which prevents the assembly of the spliceosome. The effect of pladienolide B reduced several functional parameters *in vivo* and *in vitro* through modulation of signaling pathways and imbalance of specific splicing events (Figure 4-5). Specifically, the block of SF3B1 exerts anti-tumour effects through a molecular mechanism involving survival signaling pathways (i.e. mTOR/ß-catenin/Cell-death) involving in turn, the distinct alteration of oncogenic splicing factors such as SRSF1 and imbalance of Bcl-x splicing variants resulting in decreased tumour aggressiveness and progression (Figure 6).

These results show that the SF3B1 gene serves as a diagnostic and prognostic biomarker and is simultaneously a new drug target for a better approach to glioblastoma improving patient management.

Therefore, a **first aspect** of the invention relates to a SF3B1 activity modulating agent, hereinafter referred to as the modulating agent of the invention, for the prevention, amelioration, alleviation or treatment of high-grade astrocytoma/glioma.

In this specification SF3B1 is also referred to as Splicing Factor 3b Subunit 1, SF3b155, SAP155, Splicing Factor 3b, Subunit 1, 155kDa, Spliceosome-Associated Protein 155, Splicing Factor 3B Subunit 1, Hsh155, PRPF10, Pre-MRNA Splicing Factor SF3b, 155 KDa Subunit, Pre-MRNA-Splicing Factor SF3b 155 KDa Subunit, Splicing Factor 3b, Subunit 1, 155kD, Pre-MRNA Processing 10, SAP 155, PRP10, SF3B1, Prp10, MDS. This gene encodes subunit 1 of the splicing factor 3b protein complex. Splicing factor 3b, together with splicing factor 3a and a 12S RNA unit, form the U2 small nuclear ribonucleoprotein complex (U2 snRNP). The splicing factor 3b/3a complex binds to the pre-mRNA upstream of the intron-branching site in a sequence-independent manner and can anchor the U2 snRNP component to the pre-mRNA. Splicing factor 3b is also a component of the U12-type minor spliceosome. The carboxy-terminal two-thirds of subunit 1 have 22 non-identical tandem HEAT repeats that form helical structures. Alternative splicing results in multiple transcript variants encoding different isoforms.

In the context of the present invention, *SF3B1* is also defined by a sequence of nucleotides or polynucleotides, which constitutes the coding sequence of the SF3B1 protein, and which would comprise several variants from:
(a) nucleic acid molecules encoding a polypeptide comprising the amino acid sequence of SEQ ID NO: 1,
(b) nucleic acid molecules whose complementary strand hybridizes with the polynucleotide sequence of (a),
(c) nucleic acid molecules whose sequence differs from (a) and/or (b) due to degeneracy of the genetic code,
(d) nucleic acid molecules encoding a polypeptide comprising an amino acid sequence with at least 80%, 90%, 95%, 98%, or 99% identity to SEQ ID NO: 1. wherein the polypeptide encoded by such nucleic acids possesses the activity and structural characteristics of the SF3B1 protein. Preferably, it is SEQ ID NO: 2.

SEQ ID NO: 1 
MAKIAKTHEDIEAQIREIQGKKAALDEAQGVGLDSTGYYDQEIYGGSDSRFAGYVTSIAATEL
EDDDDDYSSSTSLLGQKKPGYHAPVALLNDIPQSTEQYDPFAEHRPPKIADREDEYKKHRR
TMIISPERLDPFADGGKTPDPKMNARTYMDVMREQHLTKEEREIRQQLAEKAKAGELKVVN
GAAASQPPSKRKRRWDQTADQTPGATPKKLSSWDQAETPGHTPSLRWDETPGRAKGSET
PGATPGSKIWDPTPSHTPAGAATPGRGDTPGHATPGHGGATSSARKNRWDETPKTERDT
PGHGSGWAETPRTDRGGDSIGETPTPGASKRKSRWDETPASQMGGSTPVLTPGKTPIGTP
AMNMATPTPGHIMSMTPEQLQAWRWEREIDERNRPLSDEELDAMFPEGYKVLPPPAGYVP
IRTPARKLTATPTPLGGMTGFHMQTEDRTMKSVNDQPSGNLPFLKPDDIQYFDKLLVDVDE
STLSPEEQKERKIMKLLLKIKNGTPPMRKAALRQITDKAREFGAGPLFNQILPLLMSPTLEDQ
ERHLLVKVIDRILYKLDDLVRPYVHKILVVIEPLLIDEDYYARVEGREIISNLAKAAGLATMISTM
RPDIDNMDEYVRNTTARAFAVVASALGIPSLLPFLKAVCKSKKSWQARHTGIKIVQQIAILMG
CAILPHLRSLVEIIEHGLVDEQQKVRTISALAIAALAEAATPYGIESFDSVLKPLWKGIRQHRGK
GLAAFLKAIGYLIPLMDAEYANYYTREVMLILIREFQSPDEEMKKIVLKVVKQCCGTDGVEAN
YIKTEILPPFFKHFWQHRMALDRRNYRQLVDTTVELANKVGAAEIISRIVDDLKDEAEQYRKM
VMETIEKIMGNLGAADIDHKLEEQLIDGILYAFQEQTTEDSVMLNGFGTVVNALGKRVKPYLP
QICGTVLWRLNNKSAKVRQQAADLISRTAVVMKTCQEEKLMGHLGVVLYEYLGEEYPEVLG
SILGALKAIVNVIGMHKMTPPIKDLLPRLTPILKNRHEKVQENCIDLVGRIADRGAEYVSAREW
MRICFELLELLKAHKKAIRRATVNTFGYIAKAIGPHDVLATLLNNLKVQERQNRVCTTVAIAIVA
ETCSPFTVLPALMNEYRVPELNVQNGVLKSLSFLFEYIGEMGKDYIYAVTPLLEDALMDRDL
VHRQTASAVVQHMSLGVYGFGCEDSLNHLLNYVWPNVFETSPHVIQAVMGALEGLRVAIG
PCRMLQYCLQGLFHPARKVRDVYWKIYNSIYIGSQDALIAHYPRIYNDDKNTYIRYELDYIL
SEQ ID NO: 2

AGTTCCGTCTGTGTGTTCGAGTGGACAAAATGGCGAAGATCGCCAAGACTCACGAAGAT
ATTGAAGCACAGATTCGAGAAATTCAAGGCAAGAAGGCAGCTCTTGATGAAGCTCAAGG

AGTGGGCCTCGATTCTACAGGTTATTATGACCAGGAAATTTATGGTGGAAGTGACAGCA
GATTTGCTGGATACGTGACATCAATTGCTGCAACTGAACTTGAAGATGATGACGATGAC
TATTCATCATCTACGAGTTTGCTTGGTCAGAAGAAGCCAGGATATCATGCCCCTGTGGC
ATTGCTTAATGATATACCACAGTCAACAGAACAGTATGATCCATTTGCTGAGCACAGACC
TCCAAAGATTGCAGACCGGGAAGATGAATACAAAAAGCATAGGCGGACCATGATAATTT
CCCCAGAGCGTCTTGATCCTTTTGCAGATGGAGGAAAACCCCTGATCCTAAAATGAAT
GCTAGGACTTACATGGATGTAATGCGAGAACAACACTTGACTAAAGAAGAACGAGAAAT
TAGGCAACAGCTAGCAGAAAAAGCTAAAGCTGGAGAACTAAAAGTCGTCAATGGAGCA
GCAGCGTCCCAGCCTCCATCAAAACGAAAACGGCGTTGGGATCAAACAGCTGATCAGA
CTCCTGGTGCCACTCCCAAAAAACTATCAAGTTGGGATCAGGCAGAGACCCCTGGGCA
TACTCCTTCCTTAAGATGGGATGAGACACCAGGTCGTGCAAAGGGAAGCGAGACTCCT
GGAGCAACCCCAGGCTCAAAAATATGGGATCCTACACCTAGCCACACACCAGCGGGAG
CTGCTACTCCTGGACGAGGTGATACACCAGGCCATGCGACACCAGGCCATGGAGGCG
CAACTTCCAGTGCTCGTAAAAACAGATGGGATGAAACCCCCAAAACAGAGAGAGATACT
CCTGGGCATGGAAGTGGATGGGCTGAGACTCCTCGAACAGATCGAGGTGGAGATTCTA
TTGGTGAAACACCGACTCCTGGAGCCAGTAAAAGAAAATCACGGTGGGATGAAACACCA
GCTAGTCAGATGGGTGGAAGCACTCCAGTTCTGACCCCTGGAAAGACACCAATTGGCA
CACCAGCCATGAACATGGCTACCCCTACTCCAGGTCACATAATGAGTATGACTCCTGAA
CAGCTTCAGGCTTGGCGGTGGGAAAGAGAAATTGATGAGAGAAATCGCCCACTTTCTGA
TGAGGAATTAGATGCTATGTTCCCAGAAGGATATAAGGTACTTCCTCCTCCAGCTGGTTA
TGTTCCTATTCGAACTCCAGCTCGAAAGCTGACAGCTACTCCAACACCTTTGGGTGGTA
TGACTGGTTTCCACATGCAAACTGAAGATCGAACTATGAAAAGTGTTAATGACCAGCCAT
CTGGAAATCTTCCATTTTTAAAACCTGATGATATTCAATACTTTGATAAACTATTGGTTGA
TGTTGATGAATCAACACTTAGTCCAGAAGAGCAAAAAGAGAGAAAAATAATGAAGTTGCT
TTTAAAAATTAAGAATGGAACACCACCAATGAGAAAGGCTGCATTGCGTCAGATTACTGA
TAAAGCTCGTGAATTTGGAGCTGGTCCTTTGTTTAATCAGATTCTTCCTCTGCTGATGTC
TCCTACACTTGAGGATCAAGAGCGTCATTTACTTGTGAAAGTTATTGATAGGATACTGTA
CAAACTTGATGACTTAGTTCGTCCATATGTGCATAAGATCCTCGTGGTCATTGAACCGCT
ATTGATTGATGAAGATTACTATGCTAGAGTGGAAGGCCGAGAGATCATTTCTAATTTGGC
AAAGGCTGCTGGTCTGGCTACTATGATCTCTACCATGAGACCTGATATAGATAACATGG
ATGAGTATGTCCGTAACACAACAGCTAGAGCTTTTGCTGTTGTAGCCTCTGCCCTGGGC
ATTCCTTCTTTATTGCCCTTCTTAAAAGCTGTGTGCAAAAGCAAGAAGTCCTGGCAAGCG
AGACACACTGGTATTAAGATTGTACAACAGATAGCTATTCTTATGGGCTGTGCCATCTTG
CCACATCTTAGAAGTTTAGTTGAAATCATTGAACATGGTCTTGTGGATGAGCAGCAGAAA
GTTCGGACCATCAGTGCTTTGGCCATTGCTGCCTTGGCTGAAGCAGCAACTCCTTATGG
TATCGAATCTTTTGATTCTGTGTTAAAGCCTTTATGGAAGGGTATCCGCCAACACAGAGG

AAAGGGTTTGGCTGCTTTCTTGAAGGCTATTGGGTATCTTATTCCTCTTATGGATGCAGA
ATATGCCAACTACTATACTAGAGAAGTGATGTTAATCCTTATTCGAGAATTCCAGTCTCC
TGATGAGGAAATGAAAAAAATTGTGCTGAAGGTGGTAAAACAGTGTTGTGGGACAGATG
GTGTAGAAGCAAACTACATTAAAACAGAGATTCTTCCTCCCTTTTTAAACACTTCTGGC
AGCACAGGATGGCTTTGGATAGAAGAAATTACCGACAGTTAGTTGATACTACTGTGGAG
TTGGCAAACAAAGTAGGTGCAGCAGAAATTATATCCAGGATTGTGGATGATCTGAAAGA
TGAAGCCGAACAGTACAGAAAAATGGTGATGGAGACAATTGAGAAAATTATGGGTAATT
TGGGAGCAGCAGATATTGATCATAAACTTGAAGAACAACTGATTGATGGTATTCTTTATG
CTTTCCAAGAACAGACTACAGAGGACTCAGTAATGTTGAACGGCTTTGGCACAGTGGTT
AATGCTCTTGGCAAACGAGTCAAACCATACTTGCCTCAGATCTGTGGTACAGTTTTGTG
GCGTTTAAATAACAAATCTGCTAAAGTTAGGCAACAGGCAGCTGACTTGATTTCTCGAAC
TGCTGTTGTCATGAAGACTTGTCAAGAGGAAAAATTGATGGGACACTTGGGTGTTGTATT
GTATGAGTATTTGGGTGAAGAGTACCCTGAAGTATTGGGCAGCATTCTTGGAGCACTGA
AGGCCATTGTAAATGTCATAGGTATGCATAAGATGACTCCACCAATTAAAGATCTGCTGC
CTAGACTCACCCCCATCTTAAAGAACAGACATGAAAAAGTACAAGAGAATTGTATTGATC
TTGTTGGTCGTATTGCTGACAGGGGAGCTGAATATGTATCTGCAAGAGAGTGGATGAGG
ATTTGCTTTGAGCTTTTAGAGCTCTTAAAAGCCCACAAAAAGGCTATTCGTAGAGCCACA
GTCAACACATTTGGTTATATTGCAAAGGCCATTGGCCCTCATGATGTATTGGCTACACTT
CTGAACAACCTCAAAGTTCAAGAAAGGCAGAACAGAGTTTGTACCACTGTAGCAATAGC
TATTGTTGCAGAAACATGTTCACCCTTTACAGTACTCCCTGCCTTAATGAATGAATACAG
AGTTCCTGAACTGAATGTTCAAAATGGAGTGTTAAAATCGCTTTCCTTCTTGTTTGAATAT
ATTGGTGAAATGGGAAAAGACTACATTTATGCCGTAACACCGTTACTTGAAGATGCTTTA
ATGGATAGAGACCTTGTACACAGACAGACGGCTAGTGCAGTGGTACAGCACATGTCACT
TGGGGTTTATGGATTTGGTTGTGAAGATTCGCTGAATCACTTGTTGAACTATGTATGGCC
CAATGTATTTGAGACATCTCCTCATGTAATTCAGGCAGTTATGGGAGCCCTAGAGGGCC
TGAGAGTTGCTATTGGACCATGTAGAATGTTGCAATATTGTTTACAGGGTCTGTTTCACC
CAGCCCGGAAAGTCAGAGATGTATATTGGAAAATTTACAACTCCATCTACATTGGTTCCC
AGGACGCTCTCATAGCACATTACCCAAGAATCTACAACGATGATAAGAACACCTATATTC
GTTATGAACTTGACTATATCTTATAATTTTATTGTTTATTTTGTGTTTAATGCACAGCTACT
TCACACCTTAAACTTGCTTTGATTTGGTGATGTAAACTTTTAAACATTGCAGATCAGTGTA
GAACTGGTCATAGAGGAAGAGCTAGAAATCCAGTAGCATGATTTTTAAATAACCTGTCTT
TGTTTTTGATGTTAAACAGTAAATGCCAGTAGTGACCAAGAACACAGTGATTATATACAC
TATACTGGAGGGATTTCATTTTTAATTCATCTTTATGAAGATTTAGAACTCATTCCTTGTG
TTTAAAGGGAATGTTTAATTGAGAAATAAACATTTGTGTACAAAATGCTAATTTGTGTGTG
TTTTTTGAACATGACTTGTAAAATGCGGAACTTTGATAAAGTATTGGTTTATGTGGAATAA
GTGGCTTAATTTCATTTTCTGTCACATGGTTTATAGAAAGTAGTTAGCTGAATAAAAACTA

TATAAAGTGATGGCATCTTTGTCAAAATTCCATTGTTGTGTTAATAATGACAGCAAGAAG
AGTAGCCTCAGGGGATGTTCCCCTCAAACTAGCACAACCATTCCATCATCGTAGAAAAG
TAGCACTTTTGCTAAACTGTCTTGAATATTTTGTACTTACATAGCGCCTTTCATCTCTTGA
TTTCTCAAAATGCTTTATGAACACATTTAAAGAAAGTGGTTTAAGTCTTGTCCAACACTTG
ACAGGTCTGCTGTGTTTAGCAAGTGAGGAATTTAACTTTACTTCAAAACTGCTTTCTGCC
TATTAGGAGTGAGGATACCTAAGTAATGCTGATAGAACAGGACAATGTTGGGCTTTTCTC
CATGTTATAAGCCACTACTCAGCAATGCATCAGTAAATACCTATTCACCCACTGTATGCC
AGCCACTGTGCTTATATGCAGGGGATGCAAAGGTGCATGAGACCTGCCCTCTACCTTTA
AGAACAGTATAATGGAGAAGGGGAGACAAACCTTGTGACAGATTCCTTATGTACTGTGA
TAATACACAGTGGCAGCAGCTAAAATGTCTAGGTTTGCTTAGCTTTGTATTCAGTAATAA
TAAGTTGATCTAAGAGTTCAGCATAAACTGAATGAAATGCCATTTAATGGTAGAGGAACC
AAGCATTAAGGCAGTACTACACTTAATTTTTTAAGCAAATATGTAAAGTATATTTTCAAAC
TTTTCTAATGTTATGGCCCCAAATTTCTTAGTTTTGGCCTTTTTACTACCTATACTATTTTT
ACTGTTTTGTTTTGTCTCCATGTGGTAGTACTTCTGTGAACTCTAAAGGAAAAAAAATTC
TGCAAGCAGCATTAGTATATAACTACTACTGTAAGTAAAACTGCCTATTGACACTTTAGG
AGTTCCTGCTTCAGAAGCTTAGTTAAGAAACAGCTTGTGGCCGGGTGTGGTGGCTCATG
CCTGTAATCCCAGCACTTTGGGAGGCTGAGGCGGGCGGATCACGAGGTCAGGAGATC
GAGACCATCCTGGCTAACGCGGTGAAGTCCCGTCTACTAAAAATACAAAAATTAGCCAG
GCGTGGTGGCGGGCGCCTGTAGTCCCAGCTACTAGGGAGGCTGAGGCAGGAGAATGG
CTTGAACCCGGGAGGCGGAGCTTGCAGTGAGCCGAGATAGCGCTGCTACACTCCAGC
CTGGGCGACAGAGACTCCGTCTCAAAAAAAAAAAAAAAATTGACTTTCAACAAATTGATA
GTGAGCATTAAGGGTTTCCAAGTTGGATTTGTAACTCCTCATCATTCCTTGTATGACAAC
TTTCTGAATATATGTCACTATGTAGTAAAATTAAACACTCCAAACTCATCTTTCTGTTGTTA
GAAGTTTTCAGCGGTACTTCCATGCAACTTTAAATCTCACTGCTCTCTATGGTTGATGTC
AAATGACCTTCAGTAATGACTGAGAATTGAATACAAATAGATTACAAAGCCAAAATTTGAT
GTTAAATGACTCAGGAAATTTTAGTTGTATTTTCAATTCAAGTACTTAGTAGCCTACGTTT
GCTTGGCCTCTGGTTCTTTATGGAAAATAGGCTTTGTAGTGGCATTGTGGAGCAAAGGA
GACTGTTACACCTTAATTAACTTTTTTTACTGATGCAAATAATTTGAGGATAGAGAGGAG
GGAAGTAGTGAAAGCTATGACCTAAAACATTGGGACCAAATAGAGGCTCACAGATATTT
GGATTATTTTATGTGCTTATTATTAAATAAGGAAAGCATTTTGTGATATGTGGAAGACGCT
ATGTGAAGTTTTACCTATCTTCTCAAAGACCTTTTCTTTTGTATTTTCTTTTGGTGTTTCTT
AAAGCCAAACAAAGAAATGTTCTTAAGGAGACAGGGTGGGTTTTTCTGTGGGCCTTTGT
TGGTTTTTCTGTGGGCCATCGCCCTCTAATGGAATTGATCTCTGGCTGTTTGATTTTTTT
CATATTGTATTTTTAAAATTTGTTGTACAGTGCCCTGTGAGCACCAAGTACCACTAGATG
AATAAAACGTATTATATCTAAA

The term "activity-modulating" as used herein, refers primarily to inhibiting (decreasing) the level of SF3B1 activity. SF3B1 activity can be modulated by modifying the levels and/or activity of SF3B1, or by modifying the levels at which the gene encoding *SF3B1* is transcribed such that the activity levels of the SF3B1 protein in the cell are modulated. The modulating agents can be either agonists (substances that are capable of binding to a receptor and eliciting a response in the cell, preferably a decrease in SF3B1 activity) or antagonists (substances that not only do not activate the receptor, but block its activation by agonists). In the context of the present invention, direct inhibition is the preferred form of modulation.

In a preferred embodiment of this aspect of the invention, the modulating agent of the invention is selected from a list consisting of:
(a) an organic molecule,
(b) an RNA molecule,
(c) an antisense oligonucleotide,
(d) an antibody; or (e) a ribozyme.
or any combination thereof.

In a preferred embodiment of this aspect of the invention, the SF3B1 modulating agent of the invention is an organic molecule which is selected from the list consisting of WNT-C59, Rapamycin, IWP-2, Pilaralisib, Staurosporine, Dactolisib, GSK1904529A, Alpelisib, Crizotinib, CZC24832, Foretinib, PRIMA-1MET, PDGFRVinorelbine, Cediranib, Docetaxel, PDGFRB, Paclitaxel, ML323, TTK_3146, MK-2206, AICA, GSK2606414Flavopiridol, TAK-715, RO-3306, Linsitinib, AZD5438, NVP-ADW742, Palbociclib, Fulvestrant, THZ-2-102-1, VE-822Wee1, Mirin, Embelin, BIBR-1532, AZD5582, VX-11e, Staurosporine, KRAS, Motesanib, Lapatinib, SB505124, Cyclophosphamide, PD173074, Doxorubicin, Tivozanib, Mitomycin-C, Pazopanib, PyridostatinPD173074, Etoposide, Flavopiridol, CarmustineRO-3306, BDP-00009066, AZD5438, PAK_5339GSK343, I-BET-762, GSK591, JQ1, OF-1, PFI-1, Vorinostat, PFI3, Entinostat, EPZ5676, ACY-1215, Dacinostat, PCI-34051, Pladienolides A-G, E7107, Herboxiedene (GEX1A), FR901463, FR901465, Meayamycin, Spliceostatin A, TG003, SRPIN340, Cpd-1, Cpd-2, Cpd-3, PROTAC-O4I2, H3B-8800, Isoginkgetin, Madrasin, Tetrocarcin A, Jerantinine A, Thailanstatin A, Sudemycin K, FD-895, or any combination thereof.

In a still more preferred embodiment of this aspect of the invention, the SF3B1 modulating agent is an inhibitor, and more preferably is a compound of formula (I), hereinafter referred to as the compound of the invention: 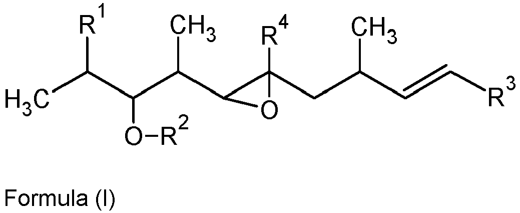 or a pharmaceutically acceptable salt thereof, wherein:
R¹ is H,OH or alkoxy;
R² is H or CH3;
R⁴ is H or CH3;
R³ is alkyl, cycloalkyl, aryl or heterocycloalkyl;

That is, one aspect of the invention relates to a compound of formula (I), or a pharmaceutically acceptable salt thereof, for the prevention, amelioration, alleviation, or treatment of high-grade astrocytoma/glioma.

The compounds described herein may inhibit an enzyme or a biochemical pathway. The terms "inhibit" and "inhibition" refer to slowing, stopping, or reversing the growth or progression of a disease, infection, condition, pathway, or group of cells. Inhibition may be greater than approximately 20%, 40%, 60%, 80%, 90%, 95%, or 99%, for example, compared to growth or progression occurring in the absence of treatment or contact.

The term "alkyl" refers to a saturated straight-chain, branched, and/or cyclic hydrocarbon ("cycloalkyl") having 1 to 20 (e.g., 1 to 10 or 1 to 4) carbon atoms. Alkyl groups having 1 to 4 carbons are referred to as "lower alkyl". Examples of alkyl groups include, but are not limited to, methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl, and dodecyl. The term "alkyl" may include "alkenyl" and "alkynyl" groups. An alkyl may be substituted with a cycloalkyl. An example of a cycloalkyl group substituted with an alkyl is 1-ethyl-4-methylcyclohexyl, and the group may be attached to a compound at any carbon atom of the group. The term "cycloalkyl" refers to a cyclic hydrocarbon having 1 to 20 (e.g., 1 to 10 or 1 to 6) carbon atoms. Examples of cycloalkyl groups include, but are not limited to, cyclopropyl, cyclobutyl, cyclopentyl, cyclohexyl, and adamantyl.

The term "alkenyl" refers to a straight-chain, branched and/or cyclic hydrocarbon having 2 to 20 (e.g., 2 to 10 or 2 to 6) carbon atoms, and including at least one carbon-carbon double bond. Representative alkenyl moieties include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2- butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

The term "alkoxy" refers to an -O-alkyl group. Examples of alkoxy groups include, but are not limited to, -OCH₃, -OCH₂CH₃, -O(CH₂)₂CH₃, -O(CH₂)₃CH₃, -O(CH₂)₄CH₃ and -O(CH₂)₅CH₃.

The term "aryl" refers to an aromatic ring or an aromatic or partially aromatic ring system composed of carbon and hydrogen atoms. An aryl group may comprise multiple rings bonded or fused together. Examples of aryl moieties include, but are not limited to, anthracenyl, azulenyl, biphenyl, fluorenyl, indan, indenyl, naphthyl, phenanthrenyl, phenyl, 1,2,3,4-tetrahydro-naphthalene, and tolyl.

The term "heteroalkyl" refers to an alkyl moiety in which at least one of its carbon atoms has been replaced by a heteroatom (e.g., N, O or S).

The term "heteroaryl" refers to an aryl moiety in which at least one of its carbon atoms has been replaced by a heteroatom (e.g., N, O or S). Examples include, but are not limited to, acridinyl, benzimidazolyl, benzofuranyl, benzoisoisothiazolyl, benzoisoxazolyl, benzoisoxazolyl, benzoquinazolinyl, benzothiazolyl, benzoxazolyl, furyl, imidazolyl, indolyl, isothiazolyl, pyrilazole, pyrilazole, pyrilazole, pyrilazole, pyrilazol, pyrilazyl, pyrilazol, pyrilazol, pyrilazol, pyrilazol, pyrilazol, pyrilazol, pyrilazol, pyrilazol, pyrilazol, pyrazinyl, pyrazinyl, pyrilazol, pyrilazol, pyrazinyl, pyrilazol, pyrazinyl, pyrilazol, pyrazinil, pyrazinil, pyrazinil, pyrazinil, pyrazinil, pyrazinil, pyrazinil, pyrazinil, pyrazinil, pyrazinil, pyrazinil, pyrazinil, pyrazinil, pyrazinil, pyrazinil, pyrazinyl, pyrilazol, pyrilazole, pyrazinyl. pyrimidyl, pyrimidyl, pyrrolyl, quinazolinyl, quinolinyl, tetrazolyl, thiazolyl and triazinyl.

The term "heteroarylalkyl" refers to a heteroaryl moiety attached to an alkyl moiety.

The term "heterocycle" refers to an aromatic, partially aromatic or non-aromatic, monocyclic or polycyclic ring or ring system composed of carbon, hydrogen and at least one heteroatom (e.g., N, O or S). A heterocycle may comprise multiple (i.e., two or more) rings fused or bonded together. Heterocycles may include heteroaryls. Examples include, but are not limited to, benzo [1,3] dioxolyl, 2,3-dihydro-benzo [1,4] dioxynyl, cinolinyl, furanyl, hydantoinyl, morpholinyl, oxetanyl, oxiranyl, piperazinyl, piperidinyl, pyrrolidinonyl, pyrrolidinyl. tetrahydrofuranil, tetrahydropyranil, tetrahydropyridinyl, tetrahydropyrimidinyl, tetrahydrothiophenyl, tetrahydrothiopyranil, tetrahydrothiopyranil and valerolactamyl.

The term "heterocycloalkyl" refers to a non-aromatic heterocycle.

The term 'substituted', when used to describe a chemical structure or chemical residue, refers to a derivative of that structure or residue in which one or more of its hydrogen atoms is substituted with a chemical residue or functional group such as, but not limited to, alcohol (e.g., hydroxyl, alkyl-OH), aldehyde, alkanoyl, alkoxyalkoxycarbonyl, alkyl (e.g, methyl, ethyl, propyl, t-butyl), alkenyl, alkynyl, alkylcarbonyloxy (-OC (O) R), amide (-C (O) NHR- or -RNHC (O) -), amidinyl (-C (NH) NHR or -C (NR) NH₂ ), amine (primary, secondary and tertiary as alkylamino, arylamino, arylalkylamino), aroyl, aryl, aryloxy, azo, carbamoyl (-NHC (O) OR- or -OC (O) NHR-), carbamyl (e.g. -CONH₂ , as well as CONH-alkyl, CONH-aryl and CONH-arylalkyl (e.g. e.g., Bn)), carbonyl, carboxyl, carboxylic acid, carboxylic acid anhydride, carboxylic acid chloride, cyano, ester, epoxide, ether (e.g. methoxy, ethoxy), guanidino, imine (primary and secondary), isocyanate, isothiocyanate, ketone, halo (F, Cl, Br or I), haloalkyl (e.g, fluoromethyl, difluoromethyl, trifluoromethyl), hemiacetal, heterocycle, nitrile, nitro, phosphodiester, sulphide, sulfonamido (e.g., SO2NH₂ ), sulfone, sulfonyl (including alkylsulfonyl, arylsulfonyl and arylalkylsulfonyl), sulfoxide, thiol (e.g., sulfhydryl, thioether), and urea (-NHCONHR-). The above groups may be elements of an R-group as described herein, including R, R¹, R², R³, R⁴ and/or R⁵. In addition, one or more of the above groups may be explicitly excluded from a definition of one of the above R-groups.

The term "pharmaceutically acceptable salts" refers to salts prepared from pharmaceutically acceptable non-toxic acids or bases including inorganic acids and bases and organic acids and bases. Suitable pharmaceutically acceptable addition salts of bases include, but are not limited to, metal salts made from aluminium, calcium, lithium, magnesium, potassium, sodium and zinc or organic salts made from lysine, N,N-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, anthranilic, benzenesulfonic, benzoic, benzoic, camphorsulfonic, citric, ethanesulfonic, formic, fumaric, furoic, galacturonic, gluconic, glucuronic, glutamic, glycolic. hydrochloric, hydrochloric, hydrochloric, isethionic, lactic, maleic, malic, malic, mandelic, methanesulphonic, mucic, nitric, pamoic, pantothenic, phenylacetic, phosphoric, propionic, salicylic, stearic, succinic, sulphanilic, sulphuric, tartaric, and p-toluenesulphonic acids. Specific non-toxic acids include hydrochloric, hydrobromic, phosphoric, sulphuric and methanesulphonic acids. Examples of specific salts therefore include hydrochloride and mesylate salts. Others are well known in the art. See, e.g., Others are well known in the art. See, e.g., Others are well known in the art. See, for example, Remington's Pharmaceutical Sciences (18th ed., Mack Publishing, Easton Pa.: 1990) and Remington: The Science and Practice of Pharmacy (19th ed., Mack Publishing, Easton Pa.: 1995).

In a more preferred embodiment of this aspect of the invention the compound of formula (I) is pladienolide B, of formula (II): 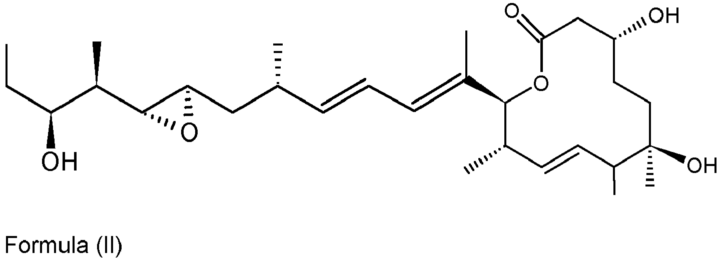 Pladienolide B, or [(2 S, 3 S, 4 E , 6 S , 7 R , 10 R ) -7,10-dihydroxy-2 - [(2 E , 4 E, 6 S ) -7 - [(2 R , 3 R ) -3 - [(2 R , 3 S ) -3-hydroxypentan-2-yl] oxiran-2-yl] -6-methylhepta-2,4-dien-2-yl] -3,7-dimethyl-12 -oxo-1-oxazacyclododec-4-en-6-yl] acetate, is a compound of CAS no: 445493-23-2.

### PHARMACEUTICAL COMPOSITION AND PHARMACEUTICAL FORM OF THE INVENTION

Another **aspect of the invention** relates to a composition comprising a modulating agent of the invention, hereinafter composition of the invention, for the prevention, amelioration, alleviation, or treatment of high-grade astrocytoma/glioma.

In a preferred embodiment of this aspect of the invention, the composition is a pharmaceutical composition. More preferably, the composition further comprises another active substance.

Another **aspect** of the invention relates to a pharmaceutical form, hereinafter pharmaceutical form of the invention, comprising a SF3B1 modulating agent of the invention, or the composition of the invention.

In this report, 'pharmaceutical form' means a mixture of one or more active ingredients with or without additives, having physical characteristics for appropriate dosage, conservation, administration, and bioavailability.

In another preferred embodiment of the present invention, the compositions and pharmaceutical forms of the invention are suitable for oral administration, in solid or liquid form. Possible forms for oral administration are tablets, capsules, syrups/syrups or solutions and may contain conventional excipients known in the pharmaceutical field, such as aggregating agents (e.g., syrup, acacia, gelatine, sorbitol, tragacanth or polyvinyl pyrrolidone), fillers (e.g., lactose, sugar, maize starch, calcium phosphate, sorbitol or glycine), disintegrants (e.g., starch, polyvinyl pyrrolidone or microcrystalline cellulose) or a pharmaceutically acceptable surfactant such as sodium lauryl sulphate. Other dosage forms may be colloidal systems, including nanoemulsions, nanocapsules, and polymeric nanoparticles.

The compositions for oral administration can be prepared by conventional galenic pharmaceutical methods, such as mixing and dispersion. Tablets can be coated by methods known in the pharmaceutical industry.

The compositions and pharmaceutical forms can be adapted for parenteral administration, such as sterile solutions, suspensions, or lyophilisates of the products of the invention, using the appropriate dosage. Suitable excipients, such as pH buffering agents or surfactants, can be used.

The above formulations can be prepared using conventional methods, such as those described in the Pharmacopoeias of different countries and in other reference texts.

As used herein, the term "active ingredient", "active substance", "pharmaceutically active substance", "active ingredient" or "pharmaceutically active ingredient" means any component that potentially provides a pharmacological activity or other different effect in the diagnosis, cure, mitigation, treatment, or prevention of disease, or affects the structure or function of the body of man or other animals. The term includes those components that promote a chemical change in the preparation of the drug and are present in the drug in a modified form intended to provide the specific activity or effect.

The term "medicament" as used herein refers to any substance used for the prevention, diagnosis, alleviation, treatment, or cure of disease in human and animals.

Administration of the compounds, compositions or pharmaceutical forms of the present invention can be performed by any suitable method, such as intravenous infusion, oral, topical or parenteral routes. Oral administration is preferred for patients' convenience.

The amount administered of a compound of the present invention will depend on the relative efficacy of the compound chosen, the severity of the disease to be treated and the weight of the patient. However, the compounds of this invention will be administered once or more times daily, for example 1, 2, 3 or 4 times daily, with a total dose between 0.1 and 1000 mg/kg/day. It is important to note that variations in dosage may be necessary, depending on the age and condition of the patient, as well as modifications in the route of administration.

The compounds and compositions of the present invention can be employed together with other drugs in combination therapies. The other drugs may be part of the same composition or of a different composition, to be administered at the same time or at different times.

### DIAGNOSTIC METHOD OF THE INVENTION

Diseases in which altered SF3B1 activity may have diagnostic potential, and in particular high-grade astrocytoma/glioma, can be detected by measuring the amount of nucleic acids (DNA and/or RNA and/or mRNA) coding for *SF3B1,* or the amount of SF3B1 protein expressed, compared to normal cells. Detection of the oligonucleotides can be done by methods well known in the state of the art (such as, but not limited to, labeled nucleotide probes, DNA-DNA or DNA-RNA hybridization, PCR amplification using labeled nucleotides, RT-PCR).

Procedures to detect SF3B1 protein expression are also well known in the state of the art, such as poly- or monoclonal antibodies, ELISA, radioimmunoassay (RIA), immunohistochemistry (IHC) and FACS (fluorescence activated cell sorting).

Therefore, in another aspect of the invention a method for obtaining data useful in the diagnosis and/or prognosis of high-grade astrocytoma/glioma is described, comprising:
(a) determining the expression of SF3B1 in a sample taken from a mammal,
(b) comparing the SF3B1 expression values obtained in (a) with standard values in healthy or diseased mammals.

### SCREENING METHOD OF THE INVENTION

Another aspect of the invention relates to a method of selecting therapeutic agents useful in the prevention, amelioration, alleviation, and/or treatment of high-grade astrocytoma/glioma, comprising:
(a) determining the activity of SF3B1 at a stated concentration of the test compound or in the absence of the test compound,
(b) determining the activity of SF3B1 at a concentration of the test compound different from that in (a).

Compounds that bind to the SF3B1 polypeptide would be identified as potential therapeutic agents against high-grade astrocytoma/glioma.

As noted above, these assays can involve the entire SF3B1 polypeptide, a biologically active fragment thereof, or a fusion protein involving all or a portion of the SF3B1 polypeptide. Determining the ability of a compound to modulate SF3B1 activity can be performed, for example, by determining the ability of SF3B1 to bind or interact with a target molecule of that compound, either directly or indirectly. It can also be an activity assay, directly or indirectly measuring the activity of SF3B1. It may also be an expression assay, directly or indirectly determining the expression of *SF3B1* mRNA or SF3B1 protein. These assays can also be combined with an *in vivo* assay measuring the effect of a test compound on symptoms of SF3B1-related diseases, and in particular high-grade astrocytoma/glioma (e.g., but not limited to, on animal models or other model systems known in the art).

The compounds to be tested used in the method for selection of therapeutic agents are not limited to low molecular weight organic molecules, proteins (including antibodies), peptides, oligogonucleotides, etc. They may be natural and/or synthetic compounds.

For example, antibodies capable of binding to an SF3B1 epitope, which can be used therapeutically, as discussed above, can also be used in immunohistochemical assays, such as Western blots, ELISAs, radioimmunoassays, immunoprecipitation assays, or other immunohistochemical assays known in the state of the art. SF3B1 polypeptides can be used to immunise an animal to obtain polyclonal antibodies. Monoclonal antibodies can also be prepared by techniques that allow the production of antibodies by cell lines in culture, including, but not limited to, hybridomas, human B-cell hybridomas. Techniques to produce chimeric, humanised or synthetic antibodies are known.

The therapeutic agents identified by the selection method described herein can be used in an animal or other model to determine the mechanism of action of the agent. Furthermore, the therapeutic agents selected by the method described herein would be used in the treatment of diseases involving SF3B1 disruption and, in particular, high-grade astrocytoma/glioma.

In another aspect of the invention, a method of selecting therapeutic agents useful in the prevention, amelioration, alleviation, and/or treatment of high-grade astrocytoma/glioma is described, comprising:
(a) determining the activity of SF3B1 at a stated concentration of the test compound or in the absence of the test compound,
(b) determining the activity of SF3B1 at a concentration of the test compound different from that in (a).

Compounds that give rise to activity other than SF3B1 would be identified as potential therapeutic agents against high-grade astrocytoma/glioma.

Throughout the description and the claims the word "comprises" and its variants are not intended to exclude other technical features, additives, components or steps. To those skilled in the art, other objects, advantages and features of the invention will be apparent in part from the description and in part from the practice of the invention. The following examples and drawings are provided by way of illustration, and are not intended to be limiting of the present invention.

### EXAMPLES OF THE INVENTION

### Reagents

Unless otherwise indicated, reagents and products were purchased from Sigma-Aldrich. Pladienolide-b (CAS 445493-23-2) (Santa Cruz Biotechnology, # sc-391691) was administered *in vitro* at 10⁻⁷ M based on a dose-response experiment and IC50 determination.

### Patients and samples

Fresh samples of high-grade astrocytomas (HGA)/glioblastoma (AIII/IV; n=22) were obtained by intracranial surgery from patients previously diagnosed with a brain tumour. Non-tumour samples were also obtained from 4 healthy brain donors by autopsy. All samples were studied histologically by expert pathologists to confirm that they were glioblastoma and non-tumour samples. Portions of each sample were flash frozen in liquid nitrogen and stored at -80°C until extraction for total RNA or paraffin-embedded samples (FFPE) for immunohistochemistry (IHC) analysis (see below). Demographic and clinical characteristics were collected for clinical correlations. This study was approved by the Ethics Committee of the Hospital Universitario Reina Sofia and was conducted in accordance with the principles of the Declaration of Helsinki. Written informed consent was obtained from all subjects included in the study.

### Bioinformatics and statistical analysis

The entire bioinformatics methodology was implemented in R 3.5 language. Obtaining and manipulating public bulk RNAseq datasets. Bulk RNAseq data from the China Glioma Genome Atlas (CGGA) (RNAseq data, n = 388; whole exome data, n = 236) and the Rembrandt microarray (GBM, n = 219; non-tumour, n = 28) were interrogated using GlioVis tools (http://gliovis.bioinfo.cnio.es) and filtered by the dplyr R package. Obtain, filter, normalize, cluster and analyze public single-cell RNAseq datasets. Adult glioblastoma single cell RNAseq data were downloaded from Single Cell Portal - Broad Institute (GSE131928; total adult cells, n = 5528) and analyzed using the Seurat package. Filtering was performed by removing cells with <200 genes and > 8000 genes along with filtering cells having a percentage of mitochondrial genes greater than 0.9, with 5123 cells being the number of cells filtered. The data were normalized using the LogNormalize method and scaled with a scale factor equal to 10000. PCA and UMAP methods were applied to perform cell clustering. The top 10 markers were used to characterize each group Processing, normalization and analysis of paired-end bulk RNAseq. Paired-end bulk RNAseq data from the EPed mouse model were aligned to the UCSC hg19 genome using STAR2. Normalization, gene association counting and differential expression analysis were achieved using Partek Flow^{®} software (Partek Incorporated, St. Louis, MO, USA).

Obtaining and manipulating public CPTAC proteomic datasets. Proteomic data from the Clinical Proteomic Tumor Analysis Consortium (CPTAC) GBM discovery study (GBM, n = 99; Non-tumor, n = 9) were downloaded from https://cptac-data-portal.georgetown.edu and filtered with the dplyr R package. Obtaining and manipulating of the Genomics of Drug Sensitivity in Cancer (GDSC) database. Resistance and sensitivity of compounds tested in 18 GBM cell lines were downloaded from https://www.cancerrxgene.org, filtered with the dplyr R package and combined with SF3B1 expression of GBM cell lines from the Broad Institute Cancer Cell Line Encyclopedia (CCLE https: //portals.broadinstitute.org/ccle).

Survival analysis. Survival groups were selected according to the cut-off points determined by the survminer R package.

Enrichment analysis. STRING database (https://string-db.org) to determine the possible functional association between several genes correlated with SF3B1 (r> ± 0.8000). Enrichment analysis was performed based on KEGG pathway analysis. The Reactome database was used to further explore relevant pathways associated with SF3B1 expression and the ggplot2 R package was used.

### Electroporated (EPed) mouse models

All experimental procedures were performed in accordance with the Cedars-Sinai Institutional Animal Care and Use Committee. Bulk RNAseq datasets of paired-end RNAseq of glioma mouse models generated by postnatal lateral ventricle electroporation after injection of plasmid DNA mixture with oncogenic mutations that had been identified as oncogenic mutations in previous models or in patient tumours (Erbb2-V664E-EGFP / Hras-G12 EGFP / Kras-G12V-EGFP tumour versus EGFP control models).

RNA isolation, real-time quantitative RT-PCR (qPCR) and customized dynamic array qPCR based on microfluidic technology. Total RNA was extracted from normal human samples and fresh tumours, followed by DNAase treatment using the AIIPrep DNA/RNA/Protein Mini Kit and RNAase-free DNAase Complex (Qiagen, no. 80004/no. 79254), respectively. Total RNA from glioblastoma cell lines was extracted with TRIzol reactive (ThermoFisher-Scientific, no. 15596026). In all cases, the concentration and purity of total RNA was assessed using the Nanodrop One Microvolume UV-Vis spectrophotometer (ThermoFisher-Scientific). For qPCR analyses, total RNA was retrotranscribed using random hexamer primers and the RevertAid RT reverse transcription kit (ThermoFisher-Scientific, no. K1691). In addition, implemented a dynamic qPCR array based on microfluidic technology (Fluidigm, # BMK-M-48.48) to determine SF3B1 expression simultaneously in human samples and cell lines using Biomark System and Fluidigm^{®} Real-Time PCR Analysis Software v.3.0.2 and data collection software v.3.1.2 (Fluidigm). Specific primers for human transcripts including SF3B1, key glioblastoma tumour markers, key point genes of selected pathways and 3 constitutive genes were specifically designed with Primer3 software. To control for variations in the efficiency of the retrotranscription reaction, the mRNA copy number of the different transcripts analyzed was adjusted by a normalization factor, calculated with the expression levels of 3 internal genes [b-actin (ACTB), hypoxanthine guanine phosphoribosyltransferase (HPRT), glyceraldehyde 3-phosphate dehydrogenase (GAPDH);] and GeNorm 3.3 software as previously reported.

### Immunohistochemical analysis

SF3B1 IHC analysis was performed on FFPE samples obtained by intracranial surgery from patients diagnosed with AAG/glioblastomas (n = 13) and control/non-pathological samples (n = 4). Specifically, rabbit polyclonal antibodies against human SF3B1 (Abcam, # ab172634) were used according to the manufacturer's instructions. Specifically, the deparaffinised sections were incubated with the antibody overnight at 4 °C. Then, ImmPRESS^{®} Anti-Mouse / Rabbit IgG PEROXIDASE (Vector Laboratories, # MP-7500-50) was used according to the supplier's recommendations. Finally, sections were developed with 3,39 diaminobenzidine (EnvisionSystem 2-KiTSolution-DAB ThermoFisher-Scientific, # 34065), in contrast to haematoxylin (# MHS128). As previously reported, pathologists performed histopathological analysis of the samples following a blinded protocol. In the analysis, 1 (+), 2 (++), 3 (+++) indicate low, moderate, and high staining intensities of the tumour region compared to the adjacent or normal control sample region.

### Cell lines

Glioblastoma cell lines (U-87 MG and U-118 MG) were obtained from the American Type Culture Collection (ATCC, # HTB-14 / # HTB 15, respectively) and cultured according to the supplier's recommendations with the number of passages being less than 15. These cell lines were pre-tested for mycoplasma contamination and often by PCR as reported above.

### Patient-derived primary glioblastoma / non-tumour brain cell culture

Fresh tissue samples were collected within 15 minutes after intracranial surgery and immediately transported to the cell culture room in cold sterile S-MEM medium (Gibco, # 11380-037) supplemented with 0.1% BSA (# A2153), 0.01% L glutamine (# G7513), 1% antibiotic-antimycotic solution (Gibco, # R01510) and 2.5% HEPES (# H3537). Fresh tissue samples were dispersed into single cells within 30 minutes using a mechanical/enzymatic protocol. Single cells were cultured on poly-L-lysine-coated tissue culture plates (# P1524-25MG) in 10% FBS (# F6765) containing D-MEM (Bl, # 06-1055-09-1A) supplemented as S - MEM medium.

### Determination of dose-response and IC50

The proliferation assay (see below) was used to develop a dose-response and IC50 determination of pladienolide-b in glioblastoma cells. The selected doses of pladienolide-b, based on previously reported *in vitro* studies, were 10⁻⁹ , 10⁻⁷ and 10⁻⁵ M measured 48 h after administration of the SF3B1 inhibitor in glioblastoma cell lines and patient-derived primary glioblastoma cell culture. Least squares regression was used as a method of adjustment to the IC50 determination.

### Measures of proliferation and migration rates

As described above, cell proliferation and migration in response to pladienolide-b treatment were analyzed using the alamar blue assay (3,000 cells/well for cell lines and 10,000 cells/well for primary cell cultures; Biosource International, # BUF012B) and the wound healing technique (150,000 U-118 MG cells/well), respectively. For the migration assay, U-118 MG cells cultured at confluence were serum deprived for 24 h to achieve cell synchronization and then wounded with a sterile 200 µl pipette tip. Wells were replaced and cells were incubated for 6 h and 24 h with medium supplemented without FBS. Wound healing was compared to the area immediately after wounding. Three images were randomly acquired along the wound per well to calculate the area using imaged software.

### Analysis of apoptosis

As previously reported, induction of apoptosis in response to pladienolide-b treatment in glioblastoma cell lines (5,000 cells/well in white-walled multiwell luminometric plates) was performed using the Caspase-Glo^{®} 3/7 assay (Promega Corporation, # G8091). Cells were serum deprived for 24 h prior to measurements which were carried out according to the manufacturer's protocols. In addition, the level of cleaved Caspase-3 protein was identified by Western blot (see below) after the pladienolide-b treatment.

### Tumour formation

This assay was performed with both glioblastoma cell lines (1000 cells/well) cultured in a Corning Costar low adherence plate (# CLS3473) using DMEM F-12 (Gibco, # 11320033) with EGF (20 ng/µl) (# SRP3027) for 10 days (refreshed every 48h, treatment with EGF and pladienolide-b). Photographs were taken to visualize and measure cell number and area after 10 days of incubation with SF3B1 inhibitor. Tumour spheres were then trypsinised to determine the number of cells per tumour sphere.

### VEGF secretion

The VEGF human ELISA kit (ThermoFisher Scientific, no. KHG0112) was used to quantify VEGF secretion in response to pladienolide-b in glioblastoma cell lines, following the manufacturer's instructions. Silenced cells (150,000 cells/well) were seeded and incubated for 24 h in FBS medium. Cells were then starved of FBS for 1 hour and incubated under the same conditions for 4 hours and 24 hours. The medium was collected, centrifuged and stored for VEGF determination.

### Western blot

To determine protein levels, cell pellets were resuspended using pre-warmed SDS-DTT sample buffer [62.5 mM Tris-HCl (# 10708976001), 2% SDS (# 71726), 20% glycerol (# 17904), 100 mM DTT (# D0632-5G) and 0.005% bromophenol blue (# B0126)] followed by sonication for 10 seconds and boiling for 5 min at 95 °C. Proteins were separated by SDS-PAGE electrophoresis with different percentage of polyacrylamide and transferred to nitrocellulose membranes (Millipore, n° 1704270). The membranes were blocked with 5% fat-free milk powders (# T145.3), in Tris-buffered saline / 0.05% -Tween 20 (# 93773) y se incubaron con el anticuerpo primario [SF3B1 (Abcam, # ab172634), Cleaved-Caspasa 3 (CST, n.o 9664), fosfo AKT (CST, n.o 4060), AKT (CST, n.o 9272), phospho -MTOR (CST, n.o 2971), MTOR (CST, n.or 2972), phospho-p70-S6K1 (CST, # 9205), p70-S6K1 (CST, # 9202), phosphoPDK1 (CST, # 3061), phosphoTP53 (SCBT, # sc-135772), TP53 (Cusabio, # CSB-MA0240771A0m), HIF1A (Novus Biological, # NB100-134) and CTNNB1 (CST, # 8480)], and their appropriate secondary antibodies [anti-rabbit (CST, # 7074) or anti-mouse (CST, # 7076)]. Proteins were developed using an enhanced chemiluminescence detection system (GE Healthcare) with stained molecular weight markers. Densitometric analysis of the bands was performed with ImageJ61 software using total protein loading (Ponceau staining, # P3504-10G) or total protein signal (in the case of AKT and ERK) as a normalization factor.

### Preclinical mouse model, Micro-CT images and haematoxylin and eosin examination

A preclinical xenograft mouse model was developed to test pladienolide-b *in vivo.* Five-week-old ATHYMFoxn1nu/nu mice (n = 6; Janvier-Labs) were subcutaneously injected with 3×10⁶ of U-87 MG cells in both flanks [in 100 µl of basal membrane extract (Trevigen, # 3432-010-01)]. Once the tumour was clearly measurable (10 days after cell inoculation), each mouse received an intratumour injection with 50 µl of pladienolide-b (10⁻⁷ M) in one flank and vehicle (1x DPBS, Sigma-Aldrich, # D1408) used as a control in another flank. Tumour growth was monitored every 2 days using a digital caliper. 20 days after injection, mice were sacrificed and each tumour was dissected, fixed, and sectioned for histopathological examination after staining with haematoxylin and eosin. Examination of the number of mitoses, vascular proliferation and necrosis was performed by experienced pathologists. Additional tumour pieces were in liquid nitrogen until RNA extraction using Trizol reagent and protein extraction using SDS-DTT buffer, as previously reported. One individual was assessed by micro-CT imaging using SkyScan1176 Bruker and a software environment to show the location of the tumour *in vivo* prior to dissection. 2D analysis along with 3D image rendering was performed using VolView 3.4 software (KitWare Inc). These experiments were conducted in accordance with the European Animal Care Regulations under the approval of the university/regional government research ethics committees.

### Detection of splicing variants in response to pladienolide-b treatment by end-point PCR

Endpoint PCR was developed using cDNA from glioblastoma cell lines, patient-derived primary glioblastoma and U-87 MG xenograft mouse models in response to pladienolide-b versus control condition to detect BCL2L1-xS and BCL2L1-xL splicing variants with only one primer pair. Primer design was performed using primers with a specific pairing in Exonlla (direct sequence) and Exonlll (reverse sequence) next to the splicing event (Figure 8f). Amplicons of different sizes were estimated and subsequently identified by agarose gel electrophoresis (BCL2L1-xS, 305 bp; BCL2L1-xL, 494 bp). The details of the endpoint PCR to detect splicing events have been optimized previously.

### Statistical analysis

Heterogeneity of variance of the data was assessed using the Kolmogorov-Smirnov test. Statistical differences were assessed by T-test, Mann-Whitney U-test or 1-way ANOVA followed by Fisher's exact test of correction. Correlations were studied using Pearson's correlation test. All statistical analyses were performed using Prism 8.0 software (GraphPad Software, La Jolla, CA, USA). P values <0.05 were considered statistically significant. Data represent median (interquartile range) or means ± SEM. * P, 0.05; ** P, 0.01; *** P, 0.001, significantly different from control conditions.

### Data availability

External bulk RNAseq data analyzed during the current study are available from GlioVis Tools (http://gliovis.bioinfo.cnio.es) and from the Single-Cell Portal-Broad Institute for single-cell data (https: //singlecell.broadinstitute.org) for single-cell RNAseq data. Data sets generated during and/or analyzed during the current study are available upon reasonable request to the corresponding author.

## Claims

1. A SF3B1 activity modulating agent for the prevention, amelioration, alleviation or treatment of HGA.

2. The modulating agent for its use according to claim 1, which is selected from a list consisting of:
(a) an organic molecule,
(b) an RNA molecule,
(c) an antisense oligonucleotide,
(d) an antibody; or (e) a ribozyme.
or any combination thereof.

3. The modulating agent for use according to any one of claims 1-2, wherein the SF3B1 modulating agent of the invention is an organic molecule which is selected from the list consisting of: WNT-C59, Rapamycin, IWP-2, Pilaralisib, Staurosporine, Dactolisib, GSK1904529A, Alpelisib, Crizotinib, CZC24832, Foretinib, PRIMA-1MET, PDGFR Vinorelbine, Cediranib, Docetaxel, PDGFRB, Paclitaxel, ML323, TTK_3146, MK-2206, AICA, GSK2606414Flavopiridol, TAK-715, RO-3306, Linsitinib, AZD5438, NVP-ADW742, Palbociclib, Fulvestrant, THZ-2-102-1, VE-822Wee1, Mirin, Embelin, BIBR-1532, AZD5582, VX-11e, Staurosporine, KRAS, Motesanib, Lapatinib, SB505124, Cyclophosphamide, PD173074, Doxorubicin, Tivozanib, Mitomycin-C, Pazopanib, PyridostatinPD173074, Etoposide, Flavopiridol, Carmustine RO-3306, BDP-00009066, AZD5438, PAK_5339, GSK343, I-BET-762, GSK591, JQ1, OF-1, PFI-1, Vorinostat, PFI3, Entinostat, EPZ5676, ACY-1215, Dacinostat, PCI-34051, Pladienolides A-G, E7107, Herboxiedene (GEX1A), FR901463, FR901465, Meayamycin, Spliceostatin A, TG003, SRPIN340, Cpd-1, Cpd-2, Cpd-3, PROTAC-O4I2, H3B-8800, Isoginkgetin, Madrasin, Tetrocarcin A, Jerantinine A, Thailanstatin A, Sudemycin K, FD-895, or any combination thereof.

4. The modulating agent for its use according to any one of claims 1-2, wherein the SF3B1-modulating agent is pladienolide-b.

5. A composition comprising a modulating agent according to any one of claims 1-4, for the prevention, amelioration, alleviation or treatment of HGA.

6. The composition according to the preceding claim, which is a pharmaceutical composition.

7. The composition according to any one of claims 5-6, further comprising another active substance.

8. A method for obtaining data useful in the diagnosis and/or prognosis of HGA, comprising:
a) determining the expression of SF3B1 in a sample taken from a mammal,
b) comparing SF3B1 expression values obtained in (a) with standard values in healthy or diseased mammals.

9. The method according to the preceding claim, wherein the mammal is a human.

10. A method for selecting therapeutic agents useful in the prevention, amelioration, alleviation, and/or treatment of HGA, comprising:
a) determining the activity of SF3B1 at a stated concentration of the compound to be tested or in the absence of said compound,
b) determining the activity of SF3B1 at a concentration of the compound to be tested different from the one in a).
